(19) Europäisches Patentamt
European Patent Office
Office européen des brevets

(11) Publication number: 0 241 291 B1

(12) # EUROPEAN PATENT SPECIFICATION

(45) Date of publication of patent specification: **02.06.93**

(51) Int. Cl.[5]: **C07D 471/04**, C07D 491/147, C07D 471/14, C07D 495/14, A61K 31/435, A61K 31/495, A61K 31/55, //(C07D471/04, 221:00,221:00),(C07D491/147, 313:00,221:00,221:00), (C07D491/147,311:00,221:00, 221:00),(C07D491/147,307:00, 221:00,221:00)

(21) Application number: **87303102.5**

(22) Date of filing: **09.04.87**

(54) **Aryl-substituted naphthyridine and pyridopyrazine derivatives.**

(30) Priority: **11.04.86 US 851068**
**20.10.86 US 921288**

(43) Date of publication of application:
**14.10.87 Bulletin 87/42**

(45) Publication of the grant of the patent:
**02.06.93 Bulletin 93/22**

(84) Designated Contracting States:
**AT BE CH DE ES FR GB GR IT LI LU NL SE**

(56) References cited:
**EP-A- 0 127 135**
**EP-A- 0 144 996**
**US-A- 4 596 809**

(73) Proprietor: **SCHERING CORPORATION**
**2000 Galloping Hill Road**
**Kenilworth New Jersey 07033(US)**

(72) Inventor: **Blythin, David John**
**487 Mountain Avenue**
**North Caldwell New Jersey 07006(US)**
Inventor: **Siegel, Marvin Ira**
**507 Maple Hill Drive**
**Woodbridge New Jersey 07095(US)**
Inventor: **Shue, Ho-Jane**
**8 Hamilton Place**
**Pine Brook New Jersey 07058(US)**
Inventor: **Smith, Sidney Randall**
**700 Spring Avenue**
**Ridgewood New Jersey 07540(US)**

(74) Representative: **Ritter, Stephen David et al**
**Mathys & Squire 10 Fleet Street**
**London EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 241 291 B1

## Description

This invention relates to tricyclic naphthyridine and pyridopyrazine derivatives and to methods for their preparation.

EP-A-0 127 135 describes fused tricyclic derivatives of naphthyridinone, pyridone and quinolone and the corresponding thiones which possess anti-allergic, antiinflammatory and cytoprotective activity. The fused tricyclic compounds disclosed in EP-A-0 127 135 have the general formula

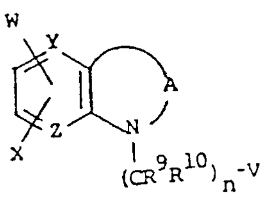

wherein A is

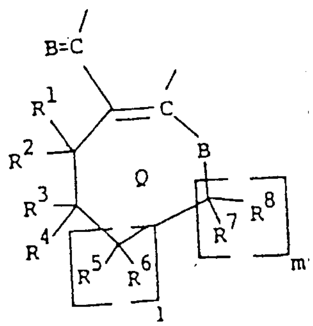

A'

or

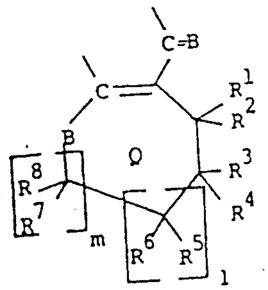

A''

wherein Y and Z represent CH or N, V, W, X, $R^9$ and $R^{10}$ represent defined substituents and A represents a fused heterocyclic ring structure containing an atom of oxygen or sulfur which is located in either the 2- or the 4- position in relation to the nitrogen marked N* in the above formula. JP-49-61,198 discloses naphthyridine derivatives of the formula:

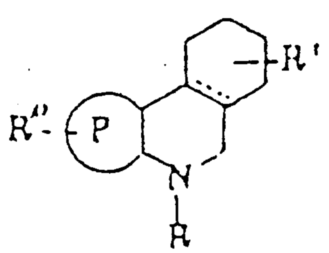

where R is hydrogen atom, a lower alkyl group, an aryl group, or an aralky group, R' and R'' are the same or different and stand for hydrogen atoms, halogen atoms, lower alkyl groups or lower alkoxy groups; P is a pyridine ring; and the dotted line means that a double bond can be either present or absent. Anti-inflammatory properties for such compounds are disclosed.

The present invention provides aryl-substituted naphthyridine and pyridopyrazine derivatives having a fused ring structure differing from that of the compounds of EP-A-0 127 135. In addition to anti-allergic, anti-inflammatory and cytoprotective activity, the compounds of the invention possess anti-hyperproliferative and immuno-suppresive activities.

One aspect of this invention comprises free bases and pharmaceutically acceptable salts thereof having the structural formula I

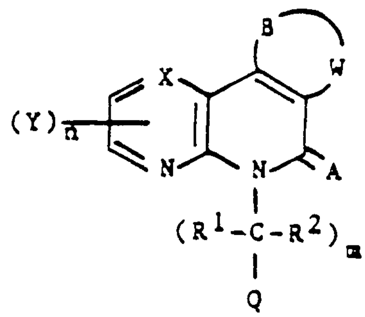

I

wherein:

X represents CH or N;

A represents O or S;

m is an integer of from 0 to 2;

n is an integer of from 0 to 2;

$R^1$ and $R^2$ are the same or different and each is independently selected from H or alkyl;

W represents a covalent bond or a group selected from -O-, $S(O)_p$-, -NH-, -N($R^4$)-, -N($COR^4$)-, and -N-($SO_2R^4$) {wherein p is an integer of from 0 to 2 and $R^4$ is alkyl};

B represents alkylene having from 2 to 8 carbon atoms, which alkylene may be optionally substituted with a group selected from -OH, -F, alkyl having from 1 to 4 carbon atoms, $-CH_2OH$, -CHO, $-CO_2H$, $-COR^3$ (wherein $R^3$ is selected from $-NHR^4$, $-N(R^4)_2$ and $-OR^4$, and $R^4$ is as defined above), and -CN, with the proviso that OH or F is not on the carbon adjacent to W when W is -O-, $S(O)_p$-,-NH-, -N($R^4$), -N($COR^4$)- or -N($SO_2R^4$). B also may represent an unsubstituted alkylene having one or more double bonds.

Q represents an aryl or an aromatic heterocyclic group which can optionally be substituted with up to 3 substituents Y as defined below; and

each Y substituent is independently selected from -OH, hydroxymethyl, alkyl, halo, $-NO_2$, alkoxy, $-CF_3$, -CN, cycloalkyl, alkynyloxy, alkenyloxy, $-S(O)_p-R^4$ {wherein $R^4$ and p are as defined above}, $-CO-R^5$ - (wherein $R^5$ represents -OH, $-NH_2$, $-NHR^4$, $N(R^4)_2$ or $-OR^4$ in which $R^4$ is as defined above), $-O-D-COR^5$ {wherein D represents alkylene having from 1 to 4 carbon atoms and $R^5$ is as defined above}, $-NH_2$, $-NHR^4$, $-N(R^4)_2$ { wherein $R^4$ is as defined above} or -NHCOH. with the proviso that when

A = O and

B = alkylene having 4 carbon atoms and

W = a covalent bond and

Q = unsubstituted $C_{6-15}$ aryl

then X represents N.

Compounds of formula I in which W is oxygen or a covalent bond are preferred. Also, A is preferably oxygen, while X is preferably CH. The group -B-W-preferably represents an alkylene or alkyleneoxy group, preferably $-(CH_2)_4-$, $-(CH_2)_5-$, $-(CH_2)_3O-$ or $-(CH_2)_4O-$. Other suitable -B-W- groups include $-CH(OH)(CH_2)_3-$, $-CH_2CH(OH)(CH_2)_2-$, $-(CH_2)CH(OH)CH_2-$, $-(CH_2)_3CHOH-$, $-CH(CH_2OH)(CH_2)_3-$, $-CH_2CH(CH_2OH)CH_2-$, $-(CH_2)_3CH(CH_2OH)-$, $-CH(OH)(CH_2)_4-$, $-CH_2CH(OH)(CH_2)_3-$, $-(CH_2)_2-CH(OH)(CH_2)_2-$, $-(CH_2)_3CH(OH)CH_2-$, $-(CH_2)_4CH(OH)-$, $-CH(CH_2OH)(CH_2)_4-$, $-CH_2CH(CH_2OH)(CH_2)_3-$, $-(CH_2)_2CH(CH_2OH)(CH_2)_2-$, $-CH_2CH(OH)-CH_2O-$, $-CH(CH_2OH)(CH_2)_2O-$, $-CH_2CH(CH_2OH)CH_2O-$, $-(CH_2)_2CH(CH_2OH)O-$, $-CH(OH)(CH_2)_3O-$, $-CH_2CH-(OH)(CH_2)_2O-$, $-(CH_2)_2CH(OH)CH_2O-$, $-CH(CH_2OH)(CH_2)_3O-$, $-CH_2CH(CH_2OH)(CH_2)_2O-$, $-(CH_2)_2CH-(CH_2OH)CH_2O-$, and $-(CH_2)_3CH(CH_2OH)O-$. The letter n preferably represents zero and m is preferably zero. Q is preferably phenyl or Y-substituted phenyl, and in the latter case each Y substituent on the Q phenyl ring is preferably selected from chloro, nitro, methoxy or trifluoromethyl. The most preferred orientation for nitro, methoxy and trifluoromethyl substituents is in the meta position.

A preferred subgenus is represented by the formula II

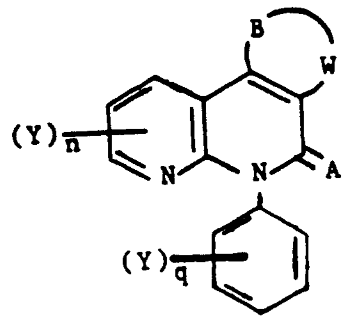

II

wherein A, B, W, n and Y are as defined above and q is 0 to 2.

When utilized herein, the terms below have the following scope:

halo - represents fluoro, chloro, bromo and iodo;

alkyl (including the alkyl portion of alkoxy) and alkylene - represent straight and branched carbon chains and, unless otherwise specified, contain from 1 to 6 carbon atoms;

alkenyloxy - represents straight and branched carbon chains having at least one carbon to carbon double bond and, unless otherwise specified, contains from 3 to 6 carbon atoms, the alkenyl group thereof being bonded to an adjacent structural element through an oxygen atom;

alkynyloxy - represents straight and branched carbon chains having at least one carbon to carbon triple bond and, unless otherwise specified, contains from 3 to 6 carbon atoms, the alkynyl group thereof being bonded to an adjacent structural element through an oxygen atom;

cycloalkyl - represents saturated carbocyclic rings having from 3 to 7 carbon atoms;

aryl - represents a carbocyclic group containing from 6 to 15 carbon atoms and having at least one benzene ring, with all available substitutable carbon atoms thereof being intended as possible points of attachment to the $(CR^1R^2)_m$ group or to the N atom if m is zero. More preferably, aryl is phenyl or Y-substituted phenyl. Suitable aryl groups include, e.g., phenyl, naphthyl, indenyl, indanyl, 3-chlorophenyl, 4-chlorophenyl, 4-fluorophenyl, etc.;

aromatic heterocyclic - represents cyclic groups having at least one O, S and/or N in the ring structure and having a sufficient number of delocalized pi electrons to provide aromatic character, with the aromatic heterocyclic groups preferably containing from 3 to 14 carbon atoms, e.g., 2-, 3- or 4-pyridyl, 2- or 3-furyl, 2- or 3-thienyl, 2-, 4- or 5-thiazolyl, 2-, 4- or 5-imidazolyl, 2-, 4-, 5- or 6-pyrimidinyl, 2- or 3-pyrazinyl, 3- or 4-pyridazinyl, 3-, 5- or 6- [1,2,4-triazinyl], 2-, 3-, 4-, 5-, 6- or 7-benzofuranyl, 2-, 3-, 4-, 5-, 6- or 7-indolyl, 3-, 4- or 5-pyrazolyl, 2-, 4- or 5-oxazolyl, etc., with all available substitutable carbon atoms thereof being intended as a possible point of attachment to the $(CR^1R^2)_m$ group or to the N atom if m is zero.

The invention in its pharmaceutical composition aspects comprises a compound having the structural formula I

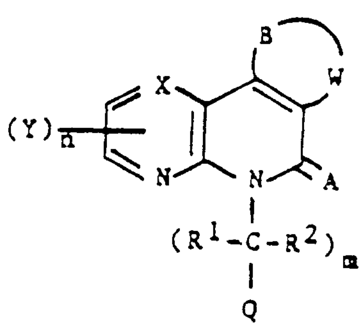

I

wherein:

X represents CH or N;

A represents O or S;

m is an integer of from 0 to 2;

n is an integer of from 0 to 2;

$R^1$ and $R^2$ are the same or different and each is independently selected from H or alkyl;

W represents a covalent bond or a group selected from -O-, $S(O)_p$-, -NH-, -$N(R^4)$-, -$N(COR^4)$-, and -N-($SO_2R^4$) {wherein p is an integer of from 0 to 2 and $R^4$ is alkyl};

B represents alkylene having from 2 to 8 carbon atoms, which alkylene may be optionally substituted with a group selected from -OH, -F, alkyl having from 1 to 4 carbon atoms, -$CH_2OH$, -CHO, -$CO_2H$, -$COR^3$ {wherein $R^3$ is selected from -$NHR^4$, -$N(R^4)_2$ and -$OR^4$, and $R^4$ is as defined above}, and -CN, with the proviso that OH or F is not on the carbon adjacent to W when W is -O-, -$S(O)_p$-,-NH-, -$N(R^4)$, -$N(COR^4)$- or -$N(SO_2R^4)$. B also may represent an unsubstituted alkylene having one or more double bonds.

Q represents an aryl or an aromatic heterocyclic group which can optionally be substituted with up to 3 substituents Y as defined below; and

each Y substituent is independently selected from -OH, hydroxymethyl, alkyl, halo, -$NO_2$, alkoxy, -$CF_3$, -CN, cycloalkyl, alkynyloxy, alkenyloxy, -$S(O)_p$-$R^4$ {wherein $R^4$ and p are as defined above}, -CO-$R^5$ {wherein $R^5$ represents -OH, -$NH_2$, -$NHR^4$, $N(R^4)_2$ or -$OR^4$ in which $R^4$ is as defined above}, -O-D-$COR^5$ {wherein D represents alkylene having from 1 to 4 carbon atoms and $R^5$ is as defined above}, -$NH_2$, -$NHR^4$, -$N(R^4)_2$ { wherein $R^4$ is as defined above} or -NHCOH.

and a pharmaceutically acceptable carrier.

The invention also includes a method for treating allergic reactions in a mammal which comprises administering an anti-allergic effective amount of the above-defined pharmaceutical composition to the mammal.

The invention in a second pharmaceutical method aspect is a method for treating inflammation in a mammal which comprises administering an anti-inflammatory effective amount of the above-defined pharmaceutical composition to the mammal.

The invention in a third pharmaceutical method aspect is a method for treating peptic ulcers in a mammal which comprises administering a cytoprotective effective amount of the above defined pharmaceutical composition to the mammal.

The invention in a fourth pharmaceutical method aspect is a method for treating hyperproliferative skin diseases, e.g. psoriasis, lichenified eczema or seborrhoeic dermatitis, in a mammal which comprises topically administering an effective amount of the above-identified pharmaceutical composition to the mammal.

The invention in a fifth pharmaceutical method aspect is a method for suppressing the immune response in a mammal which comprises administering to a mammal in need of such treatment an immunosuppressing effective amount of the above defined pharmaceutical composition.

Another aspect of the invention is a method for manufacturing a composition of formula I said method characterized by:

a) treating a compound of formula IV

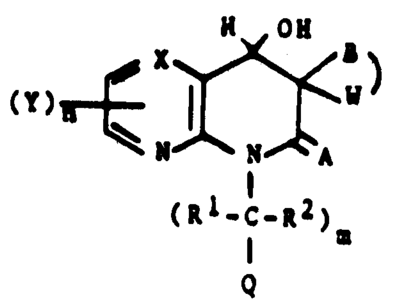

with a superacid having a Hammett activity function of less than minus 12;

b) contacting a compound of the formula

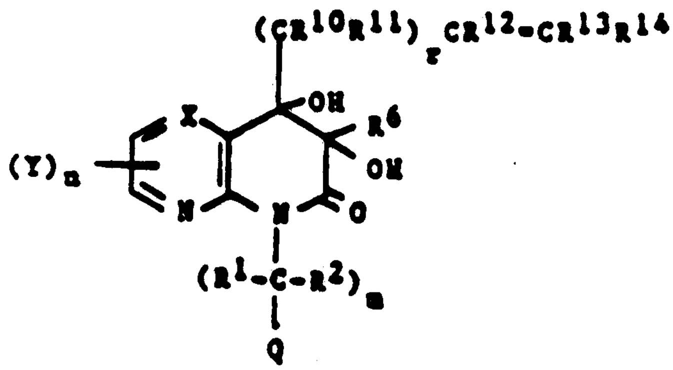

XI.

with a strong organic or inorganic acid to produce compound I;
c) contacting a compound of the formula

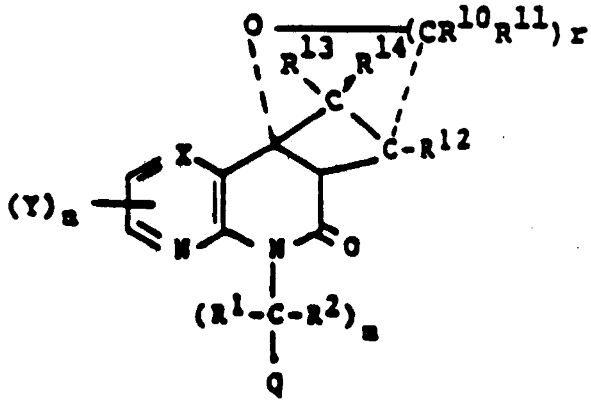

XIV.

with a strong base to form a compound of formula I; or
d) contacting a compound of the formula

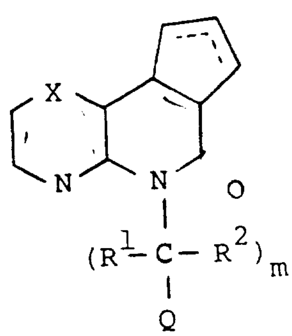

with a reducing agent to produce a compound of the formula

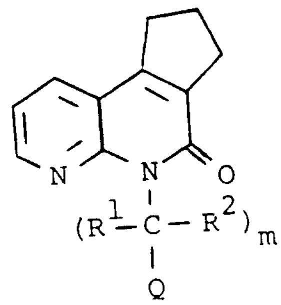

wherein X, A, m, n, W, B, Q, Y, $R^1$, $R^2$ are as previously defined and
wherein $R^6$ is an alkyl group having from 2 to 8 carbon atoms;

$R^{10}$, $R^{11}$, $R^{12}$, $R^{13}$ and $R^{14}$ independently are H or an alkyl having from 1 to 4 carbon atoms;
r is 0 or 1.

The compounds of formula I contain a $-(CR^1R^2)_m$-substituent wherein each $R^1$ group and each $R^2$ group may vary independently. Thus, for example, when m equals 2 the following patterns of substitution (wherein hydrogen and $CH_3$ are used to represent any substituent, $R^1$ or $R^2$) are contemplated: $-C(CH_3)_2CH_2-$, $-CH_2C(CH_3)_2-$, $-CH_2CH(CH_3)-$, $CH(CH_3)CH_2-$ or $-(C(CH_3)H)_2-$.

As noted above, the compounds of the invention may include one or two Y substituents on the fused ring system. Also, the Q group may include up to three Y substituents depending upon the available sites for substitution. In compounds where there is more than one such Y substituent, they may be the same or different. Thus, compounds having combinations of different Y substituents are contemplated within the scope of the invention. Examples of suitable Y substituents include hydroxy, methyl, chloro, bromo, methoxy, cyclohexyl, allyloxy, 2-propynyloxy, methylthio, methylsulfonyl, carboxy, acetoxy, N-methylaminocarbonyl, acetoxymethoxy, acetylamino or methylsulfonylamino.

Compounds of the invention of formulas I and II can exist in unsolvated as well as solvated forms, including hydrated forms, e.g., hemihydrate. In general, the solvated forms, with pharmaceutically acceptable solvents such as water, ethanol and the like are equivalent to the unsolvated forms for purposes of this invention.

Certain compounds of the invention may exist in isomeric forms. The invention contemplates all such isomers both in pure form and in admixture, including racemic mixtures.

Certain compounds of the invention also form pharmaceutically acceptable salts with organic and inorganic acids, e.g., the pyrido- or pyrazino- nitrogen atoms may form salts with strong acid while compounds having basic Y substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous sodium hydroxide, potassium carbonate, ammonia and sodium bicarbonate. The free base forms differ from their respective salt forms somewhat in certain physical properties, such as solubility in polar solvents, but the salts are otherwise equivalent to their respective free base forms for purposes of the invention.

Also, some compounds of this invention are acidic, e.g., when Y is OH, and can form salts with inorganic and organic bases.

The compounds of this invention may be synthesized from the corresponding 3-spiro-4-keto analogues of formula III below,

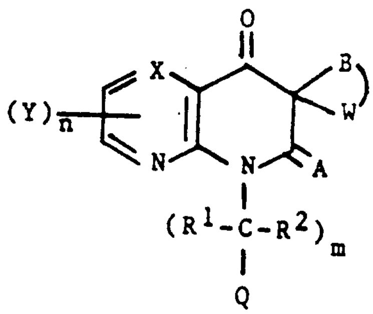

III

which may be synthesized following the procedures described in U.S Patent No. 4,762,564 issued March 24, 1987 and in EPO Publication No. 0 144 996, published June 19, 1985. Alternative synthetic routes for the synthesis of these starting materials and substitutional variants thereof may be accomplished by those skilled in the art.

This process involves a selective reduction of the 4-keto group of the compound of formula III followed by dehydration and rearrangement in the presence of a strong organic or inorganic acid. In particular, the compound of formula III is selectively reduced at the 4 position using a reducing agent capable of reducing ketones in the presence of an amide function in an acidic medium, e.g., a hydride reducing agent, to produce a compound of formula IV

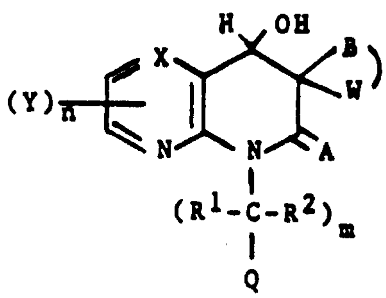

IV.

Examples of suitable reducing agents for this step are sodium cyanoborohydride and tert-butylamine borane. For a discussion of such selective reducing agents, see, for example, Herbert C. Brown, Boranes in Organic Chemistry, Cornell University Press, Ithaca and London, 1972. The reactions may be performed with cooling, with heating or at room temperature, as appropriate for the particular material being treated, e.g. at about 0°C to about 40°C. Typically, the reaction is essentially complete in several minutes, but some reactions take several days to obtain maximum yield.

Suitable solvents are those which are capable of dissolving the starting materials and which do not react with the reducing agent to make the solution basic, of which aqueous alcohol and aqueous tetrahydrofuran in combination with a weak mineral or carboxylic acid, such as acetic acid, are examples.

The compounds according to structural formula IV are treated, either in their impure state or after suitable purification using techniques well known to those versed in the art, e.g., chromatography, with a strong organic or inorganic acid such as $H_2SO_4$, methanesulfonic acid, Eaton's reagent or polyphosphoric acid, or strongly acidic salts such as $NaHSO_4$. Superacids having a Hammett acidity function of less than about minus 12, i.e., minus 13 or minus 14, provide particularly advantageous results in this process. Suitable superacids include trifluoromethanesulfonic acid, $HF/BF_3$ or $CH_3SO_3H/BF_3$. This measure of acidity is defined in Hammett, Louis P., and Deyup, Alden J., Journal of the American Chemical Society, Vol. 54, 1932, p. 2721. The time and temperature of the reaction can vary depending on the acid employed. For example, with $CF_3SO_3H$ as the acid the temperature is generally in the range of from about room temperature (e.g., 25°C.) or below to about 150°C. Lower temperature (e.g., from about -78°C. to about 25°C.) may also be employed with for example $HF/BF_3$. The acid is also generally used in excess, e.g., in an amount of from about 1.5 to about 30 equivalents. While not wishing to be bound to a specific mechanism, it appears that this treatment causes rearrangement of the spiro ring to form the ring

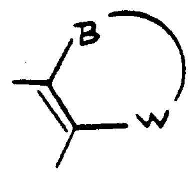

with the elimination of water, thus providing compounds of formula I. No diluent is required, but an inert cosolvent, such as a halohydrocarbon, e.g., methylene chloride, may be used.

Certain compounds of the invention can be prepared by an alternative reaction scheme employing as starting materials compounds of the formula V

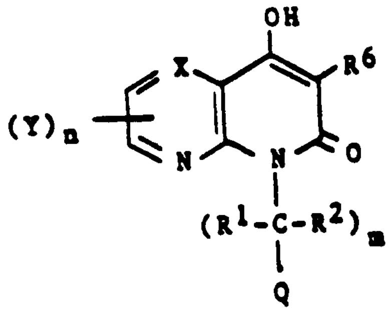

V

wherein $R^1$, $R^2$, Q, X, Y, m and n are as defined above and $R^6$ is an alkyl group having from 2 to 8 carbon atoms. Such compounds can be prepared as described in U.S. Patent No. 4,492,702 or by reaction of a compound of the formula VI with a compound of formula VII

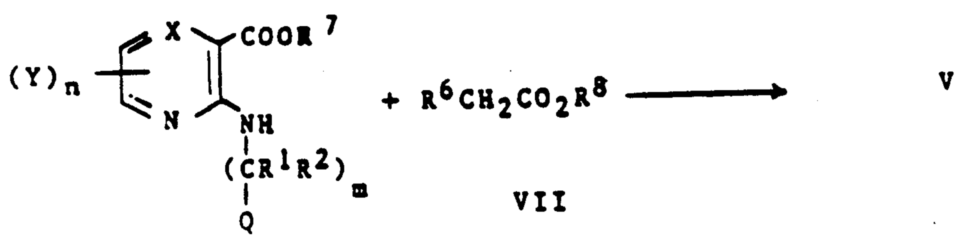

VI

VII

wherein $R^1$, $R^2$, $R^6$, Q, X, Y, m and n are as defined above and $R^7$ and $R^8$ are the same or different and are alkyl of from 1 to 8 carbon atoms.

In this alternative method, the compound of formula V is first reacted with an electrophilic halogenating agent, e.g., $Br_2$, $I_2$ + KI or, ICl, to produce a compound of formula VIII:

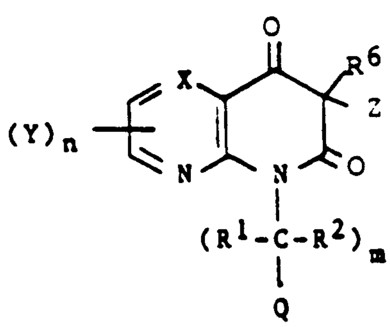

VIII

wherein Z represents halo. This reaction can be performed in an inert solvent and at any suitable temperature, preferably, at room temperature or below.

The compound of formula VIII is subjected to a nucleophilic displacement with an alcohol, e.g., an alkanol such as methanol or an aralkanol such as benzyl alcohol. For example, the compound of formula VIII may be reacted with 1,8-diazobicyclo[5.4.0]undec-7-ene [DBU] and an alcohol, with the alcohol group replacing the halide group to produce a compound of formula IX

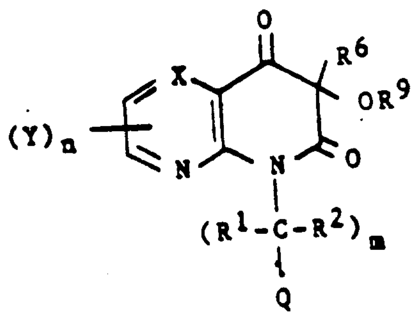

IX

wherein $R^9$ is the residue of the alcohol, e.g. alkyl group such as methyl or aralkyl group such as benzyl. This reaction may be conducted in an inert solvent and at any suitable temperature, again preferably at room temperature or below.

The compound of formula IX is reacted with a compound of the formula M-$(CR^{10}R^{11})r$ $CR^{12}=CR^{13}R^{14}$ {wherein M is an alkali metal such as Li or is Mg-Z where Z is a halo group; $R^{10}$, $R^{11}$, $R^{12}$, $R^{13}$ and $R^{14}$ may be the same or different and each is selected from H or alkyl having from 1 to 4 carbon atoms; and r is 0 or 1} to produce a compound of formula X

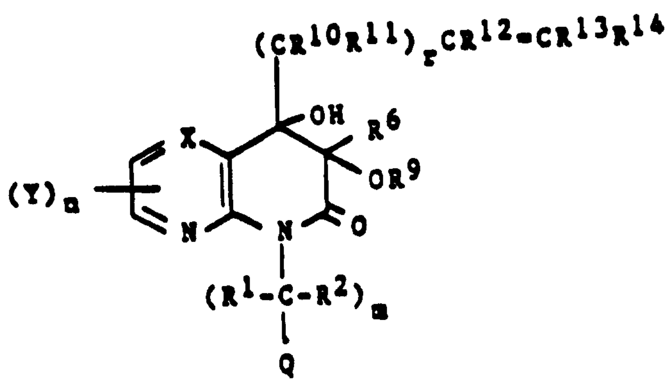

X.

This reaction is performed under conventional conditions, e.g., in an inert solvent such as THF or diethyl ether and at any suitable temperature up to reflux.

The $R^9$ group is then removed with an ether cleaving reagent, e.g., $CF_3SO_3H$, $BBr_3$, $C_2H_5SH$ and $AlCl_3$, $K^+C_2H_5S^-$ in DMF, $Na^+C_2H_5S^-$ in DMF, etc., under conventional reaction conditions for such reactions to produce a compound of formula XI

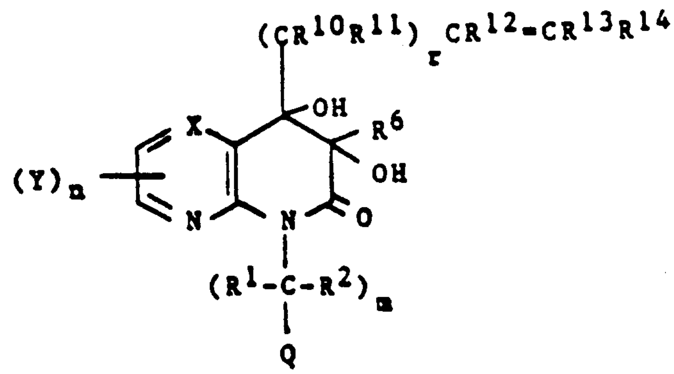

XI.

The $R^6$ alkyl group is eliminated and a cyclization/dehydration is effected by use of a strong organic or inorganic acid, such as $CF_3SO_3H$, polyphosphoric acid, Eaton's reagent, $P_2O_5$ in $CHCl_3$, etc., to produce an alkene from the $R^6$ group and a compound of formula XII

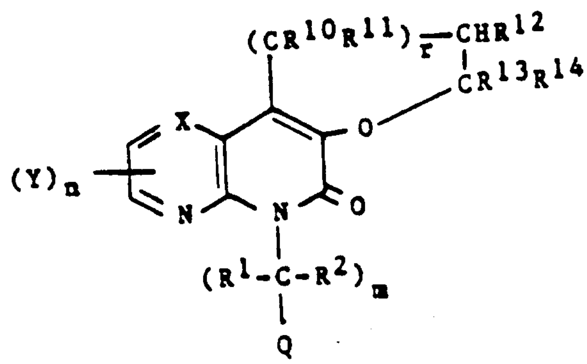

XII.

When r is 1, the following isomer may also result:

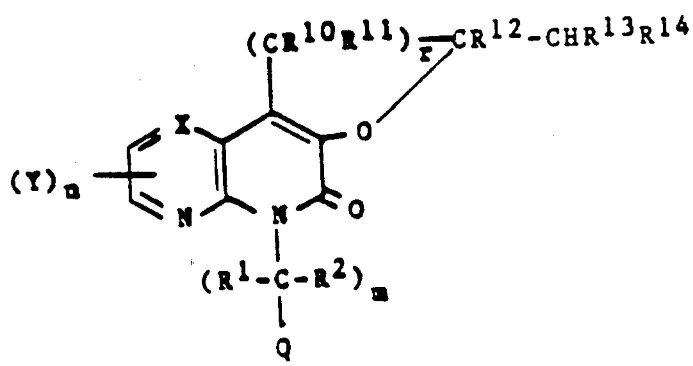

XIIa.

This reaction can be performed neat or with an inert solvent and at any suitable temperature, preferably at room temperature or below.

In another method similar to that described in C. Keneko, T. Naito and M. Somei, J.C.S. Chem. Comm., 804 (1979), a compound of formula XIII

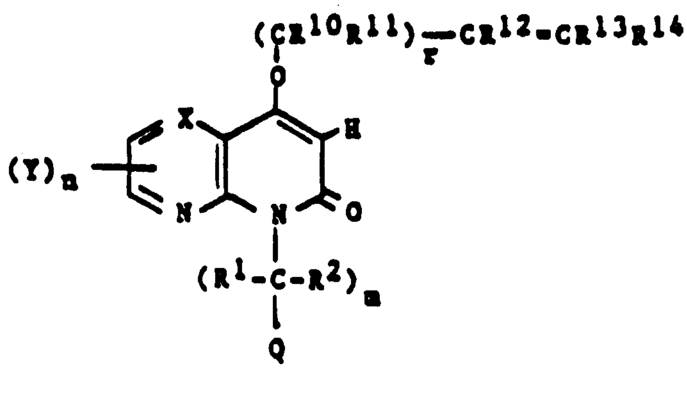

XIII

{wherein $R^1$, $R^2$, $R^{10}$, $R^{11}$, $R^{12}$, $R^{13}$, $R^{14}$, Q, X, Y, m, n and r are as defined above} can be irradiated with ultraviolet radiation, e.g., about 3000Å, to produce a compound of formula XIV

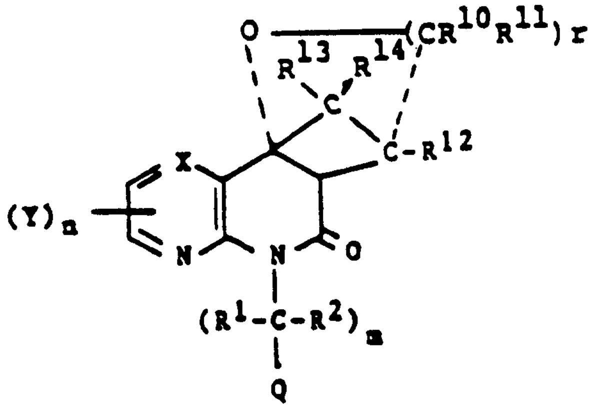

XIV.

The compound of formula XIII is preferably in an inert solvent, e.g., an alcohol such as methanol, or methanol/$CH_2Cl_2$, and the reaction mixture may be cooled during irradiation, if necessary. The starting compounds of formula XIII can be prepared by the methods described in U.S. Patent No. 4,492,702.

The compound of formula XIV is reacted with a strong base such as a salt of an alcohol, e.g., a sodium or potassium alkoxide such as $NaOCH_3$, to produce a compound of formula XV

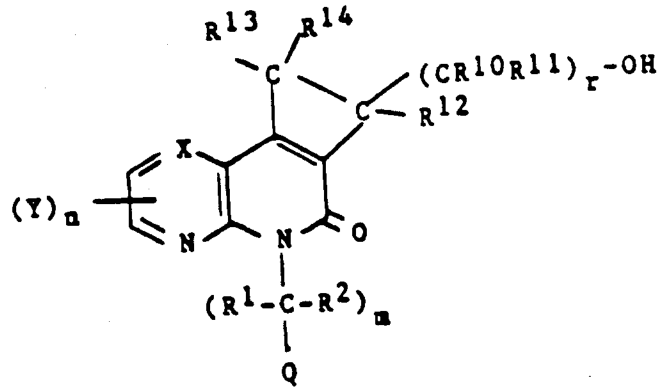

XV.

This last reaction can be conducted in an inert solvent, e.g., an alcohol such as methanol, and at any suitable temperature, preferably at about 75° to about 125°C.

The compounds of this invention wherein A is sulfur may be obtained by treating the purified 2-carbonyl compound of formula I with thiating reagents well known in the art. Lawesson's Reagent {2,4-bis(4-methoxyphenyl-1,3-dithia-2,4-diphosphetane-2,4-disulfide} or one of its analogs, in toluene, or phosphorus pentasulfide in pyridine is suitable for this purpose.

For preparing pharmaceutical compositions from the compounds described by this invention, inert, pharmaceutically acceptable carriers are admixed with the active compounds. The pharmaceutically acceptable carriers may be either solid or liquid. Solid form preparations include powders, tablets, dispersible granules, capsules, cachets and suppositories. A solid carrier can be one or more substances which may also act as diluents, flavoring agents, solubilizers, lubricants, suspending agents, binders or tablet disintegrating agents, it may also be an encapsulating material.

Liquid from preparations include solutions, suspensions and emulsions. As an example may be mentioned water or water-propylene glycol solutions for parenteral injection. Liquid preparations can also be formulated in solution or suspension in aqueous polyethylene glycol solution.

Formulations for topical application may include creams, aerosols, sprays, dusts, powders, lotions and ointments which are prepared by combining an active ingredient according to this invention with conventional pharmaceutical diluents and carriers commonly used in topical dry, liquid, cream and aerosol formulations. Ointments and creams may, for example, be formulated with an aqueous or oily base with the addition of suitable thickening and/or gelling agents.

Lotions may be formulations with an aqueous or oily base and will, in general, also include one or more of the following, namely, stabilizing agents, emulsifying agents, dispersing agents, suspending agents, thickening agents, coloring agents or perfumes.

Powders may be formed with the aid of any suitable powder base, e.g. talc, lactose or starch. Drops may be formulated with an aqueous base or non-aqueous base also comprising one or more dispersing agents, suspending agents or solubilizing agents.

The topical pharmaceutical compositions according to the invention may also include one or more preservatives or bacteriostatic agents, e.g., methyl hydroxybenzoate, propyl hydroxybenzoate, chlorocresol or benzalkonium chlorides.

The topical pharmaceutical compositions according to the invention may also contain other active ingredients such as antimicrobial agents, particularly antibiotics, anesthetics, analgesics and antipruritic agents.

Also included are solid form preparations which are intended to be converted, shortly before use, to liquid form preparations for either oral or parenteral administration. Such liquid forms include solutions, suspensions and emulsions.

The compounds of the invention may also be deliverable transdermally for systemic distribution. The transdermal compositions can take the form of creams, lotions and/or emulsions and can be included in a transdermal patch of the matrix or reservoir type as are conventional in the art for this purpose.

Preferably, the pharmaceutical preparation is in unit dosage form. In such form, the preparation is subdivided into unit doses containing appropriate quantities of the active components.

The compounds of this invention may be employed as anti-allergy agents in the treatment of, for example, asthma, allergic or seasonal rhinitis, and/or chronic bronchitis. The anti-allergy method of this

invention is identified by tests which measure a compound's inhibition of anaphylactic bronchospasm in sensitized guinea pigs having antigen induced broncho-constriction.

In one such test procedure, male Hartley guinea pigs (250-300 g) are sensitized with 5 mg ovalbumin injected i.p. and 5 mg injected s.c. in 1 ml saline on day 1 and 5 mg ovalbumin injected i.p. on day 4. The sensitized animals are used 3-4 weeks later at which time they weigh 450-500 g.

The sensitized guinea pigs are fasted overnight and the following morning are anesthetized with 0.9 ml/kg i.p. of dialurethane (0.1 g/ml diallybarbituric acid, 0.4 g/ml ethylurea and 0.4 g/ml urethane). The trachea are cannulated and the animals are ventilated by a Harvard® rodent respirator at 50 strokes/minute with a stroke volume of 5 ml. A side arm to the tracheal cannula is connected to a pressure transducer (Harvard) to obtain a continuous measure of intratracheal pressure which is recorded on a polygraph (Harvard). The jugular vein is cannulated for the i.v. administration of substances. The animals are challenged with antigen (0.5% ovalbumin) as an aerosol generated from a DeVilbiss® Model 65 ultrasonic nebulizer and delivered through the tracheal cannula for 30 seconds. Bronchoconstriction is measured as the peak increase in intratracheal pressure occurring within 5 minutes after antigen challenge.

The sensitized guinea pigs are injected i.v. with 1 mg/kg propranolol, 5 mg/kg indomethacin and 2 mg/kg mepyramine given together in a volume of 1 ml/kg. Fifteen minutes later the animals are challenged with nebulized ovalbumin. Test compounds are administered orally 2 hours before challenge with ovalbumin. Suppression of anaphylactic bronchospasm is expressed as a percent inhibition of the peak increase in intratracheal pressure by comparison to a vehicle-treated control group.

Four compounds of the invention tested in the above procedure were found to inhibit anaphylactic brochospasm as indicated in Table I below:

Table I

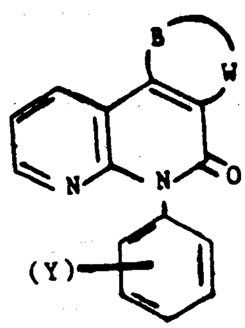

| Compound No. | Y | -B-W- | $ED_{50}$ (p.o.)-in anaphylactic bronchospasm test |
|---|---|---|---|
| 1 | m-Cl | $-(CH_2)_4-$ | 2 mg/kg |
| 2 (hemihydrate) | H | $-(CH_2)_4-$ | 2 mg/kg |
| 3 | m-Cl | $-(CH_2)_4-O-$ | 5 mg/kg |
| 4 | H | $-(CH_2)_4-O-$ | 1 mg/kg |

These compounds were also found to inhibit allergen-induced histamine release from guinea pig and human sensitized tissue.

The compounds are effective non-adrenergic, non-anticholinergic, antianaphylactic agents. When administered orally they are active at doses from about 0.5 to 25 mg/kg of body weight, preferably 0.5 to 10

mg/kg; when administered parenterally, e.g., intravenously, the compounds are active at dosages of from about 0.1 to 5 mg/kg body weight preferably 0.1 to 2.5, and when administered by inhalation (aerosol or nebulizer) the compounds are active at dosages of about 0.1 to 5 mg per puff, one to four puffs may be taken every 4 hours.

The compounds of this invention are also useful for the treatment of inflammation. Thus, they are useful in the treatment of arthritis, bursitis, tendonitis, gout and other physical conditions characterized by inflammation. The anti-inflammatory use of the compounds of this invention may be demonstrated by the Reversed Passive Arthus Response Technique, as described below.

Reversed Passive Arthus Response (RPAR) Animals, Materials and Methods

Male Lewis inbred albino rats weighing 180-200 grams obtained from Charles River Breeding Laboratories are used in these experiments. The rats are housed 3 animals/cage and food and water are allowed ad libitum. The animals are numbered 1-3 in each cage and color marked for identification purposes.

Drug and Reagent Preparation

All reagents and drugs are prepared just prior to the study. Crystallized and lyophilized bovine serum albumin (BSA), available from Sigma Chemical Company, is solubilized without shaking in cold, sterile, pyrogenfree saline (10 mg/ml). Lyophilized anti-bovine serum albumin (IgG fraction), obtained from Cappel Laboratories, is suspended in sterile distilled water and diluted with cold, pyrogen-free saline (PFS) just prior to use. The final concentration of anti-bovine serum albumin is 0.5. mg/ml of PFS. Both BSA and anti-BSA solutions are iced during use. Drugs are suspended or solubilized in an aqueous solution of methyl cellulose (MC) with an homogenizer just prior to administration.

Drug Administration and Induction of Inflammation

Groups of animals (6/group) are dosed with drug in MC by gavage once daily for 3 days. The last dose is administered one hour prior to sensitization with BSA. Controls are given MC alone and a drug-standard is usually included in each assay for verification purposes. Drugs are prepared and diluted so as to provide a dose for a 200 gram animal which is equivalent to the mg/kg dose for each experiment. Thus each rat receives an oral dose in a volume of approximately 2.0 cc. One hour after the last dose the animals are lightly anesthetized with ether and "sensitized" by injection of 0.2 ml of PFS containing 1.0 mg of BSA into the penile vein. One hour later, the animals are "challenged" in the right rear paw with subplantar injections of 0.2 ml of ml of PFS containing 0.1 mg of anti-BSA. Immediately after the subplantar injection, the right paw is dipped (up to the lateral maleolus) into the mercury well of a plethysmograph. The volume of mercury displaced is converted to weight and recorded. This value is considered to be the control reading for the animal. Paw volumes are subsequently recorded with a plethysmograph during the development of the inflammation at 2 and 4 hours post-challenge.

Results

Results are expressed by the change in paw volume (Δ paw volume) from the control reading for each animal to that recorded 2 and 4 hours post-challenge. All drug treated groups are compared to the MC control for significant differences with an analysis of variance. Differences from control in drug-treated groups are expressed as percent change from control in Table II.

TABLE II

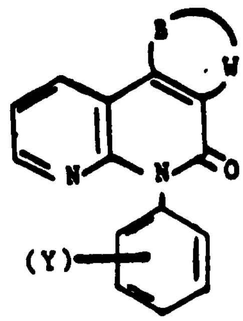

| Compound No. | Y | -B-W- | Dose (p.o) | Percent Inhibition vs. Control | |
|---|---|---|---|---|---|
| | | | | at 2 hrs. | at 4 hrs. |
| 1 | m-Cl | $-(CH_2)_4-$ | 25 mg/kg | 64 | 30 |
| 2 (hemihydrate) | H | $-(CH_2)_4-$ | 25 mg/kg | 75 | 29 |
| 4 | H | $-(CH_2)_4O-$ | 25 mg/kg | 74 | 25 |

On the basis of the test results, an oral dosage range of from about 5 mg/kg of body weight per day to about 50 mg/kg of body weight per day in divided doses taken at about 4 hour intervals is recommended. The dosage to be administered and the route of administration depends upon the particular compound used, the age and general health of the patient and the severity of the inflammatory condition. Thus, the dose ultimately decided upon must be left to the judgment of a trained health-care practitioner.

The compounds of this invention are also useful in the treatment of peptic ulcers. They display chemotherapeutic activity which enables them to relieve the symptoms of peptic ulcer disease and stress ulceration, and to promote healing of gastric and/or duodenal ulcers. The antiulcer activity of the compounds of this invention is identified by standard tests which measure the cytoprotective effect in rats, e.g., by inducing gastrointestinal damage with ethanol prior to administering a compound of the invention. The compounds may be used as conjunctive therapeutic agents for coadministration with such anti-inflammatory/analgesic agents as aspirin, indomethacin, phenylbutazone, ibuprofen, naproxen, tolmetin and other agents. The compounds of this invention prevent the untoward side effects of irritation and damage to the gastrointestinal tract caused by such agents.

In the treatment of peptic ulcer disease, and the prevention and treatment of drug-induced gastric ulceration, the active compounds of this invention can be administered in unit dosage forms such as tablets, capsules, pills, powders, granules, sterile parenteral solutions or suspensions, suppositories, mechanical delivery devices, e.g. transdermal. The compounds of this invention may be administered at doses of about 0.05 - 50 mg/kg of body weight per day. Preferably the total dosages are administered in 2-4 divided doses per day.

The compounds of formula I are useful in the treatment of hyperproliferative skin disease, e.g., psoriasis, which utility may be demonstrated by the Arachidonic Acid Mouse Ear Test as described below.

Arachidonic Acid Mouse Ear Test, Materials and Methods

Charles River, female, CD, (SD) BR mice, 6 weeks old, are caged 8/group and allowed to acclimate 1-3 weeks prior to use.

Arachidonic acid (AA) is dissolved in reagent grade acetone (2 mg/.01 ml) and stored at -20 °C for a maximum of 1 week prior to use. Inflammatory reactions are induced by applying 10 $\mu l$ of AA to both surfaces of one ear (4 mg total).

Test drugs are dissolved in either reagent grade acetone or aqueous ethanol (only if insoluble in acetone) at the same doses selected by Opas et al., Fed. Proc. 43, Abstract 2983, p. 1927 (1984) and Young et al., J. Invest. Dermatol. 82, pp 367-371 (1984). These doses are employed to ensure maximum responses and to overcome any difference in topical absorption which could occur with any drug applied in an aqueous ethanol vehicle. The test drug is applied 30 minutes prior to challenge with AA.

The severity of the inflammation is measured as a function of increased ear weight. A 6 mm punch biopsy is removed 1 hour after AA challenge and weighed to the nearest 0.1 mg. Mean ± standard error and all possible comparisons are made via Duncan's Multiple Range Statistic.

As a result of the topical administration of a compound of formula I, a remission of the symptoms of the psoriatic patient, in most cases, can be expected. Thus, one affected by psoriasis can expect a decrease in scaling, erythema, size of the plaques, pruritus and other symptoms associated with psoriasis. The dosage of medicament and the length of time required for successfully treating each individual psoriatic patient may vary, but those skilled in the art of medicine will be able to recognize these variations and adjust the course of therapy accordingly.

Included within the invention are preparation for topical application to the skin whereby the compounds having structural formula I are effective in the treatment and control of skin diseases characterized by rapid rates of cell proliferation and/or abnormal cell proliferation, e.g. psoriasis.

In a preferred method of carrying out the invention, a pharmaceutical formulation comprising a compound of formula I together with a non-toxic, pharmaceutically acceptable topical carrier, usually in concentrations in the range of from about 0.001 percent to about 10 percent, preferably from about 0.1 percent to about 5 percent, is applied several times daily to the affected skin until the condition has improved. Topical applications may then be continued at less frequent intervals (e.g. once a day) to control mitosis in order to prevent return of severe disease conditions.

The compounds of formula I also are useful in the treatment of autoimmune and other immunological diseases including graft rejection in which T cell proliferation is a contributing factor to the pathogenesis of disease. The compounds of formula I which are most effective in treating immunological diseases may not necessarily be the same compounds which are most effective as anti-inflammatory and/or anti-allergy agents.

The effectiveness of compounds of formula I as immunosuppressing agents may be demonstrated by the following tests which involve the inhibition of T cell functions using these compounds.

GRAFT VS. HOST REACTION (GVHR)

To induce a GVHR, C57 B1/6XA/J(B6AF1) male mice were injected intravenously with parental (C57B1/6J) spleen and lymph node cells. The compound 1,2,3,4-tetrahydro-7-phenyl-oxepino[2,3-c][1,8]-naphthyridin-6(7H)-one (Compound A) was then administered orally for 10 days beginning on the day prior to the cell transfer. On the day following the last treatment, the animals were sacrificed, and their spleens were excised and weighed. The enlargement of the spleen of the host is a result of a GVHR. To some extent it is the host's own cells which infiltrate and enlarge the spleen although they do this because of the presence of graft cells reacting against the host. The amount of spleen enlargement, splenonmegaly, is taken as a measure of the severity of the GVHR.

In carrying out the GVHR the animal in the experimental group is injected with parental cells, cells of the same species but of different genotyne, which cause a weight increase of the spleen. The animal in the control group is injected with syngeneic cells, genetically identical cells which do not cause a weight increase of the spleen. The effectiveness of Compound A adminstered to the mice in the experimental group is measured by comparing the spleen weight of the untreated and treated GVH animal with that of the syngeneic control. Compound A reduced spleen weight by 61% as compared to the untreated animals at a dose of 10 mg/kg.

SPLENIC ATROPHY

The immunosuppressive activity of the compounds may also be shown by a decrease in spleen weight after dosing $BDF_1$ mice orally with the drug for seven (7) consecutive days. The mice are sacrificed on the eighth day. The percent decrease in spleen weight is measured for each dosage level. In this procedure (Compound A) provided a 30% spleen weight decrease at a dosage level of 100 mg/kg.

As mentioned in Serial No. 851,068, filed April 11, 1986, the subject compounds possess anti-allergy and anti-inflammatory activities. For example, Compound A has an $ED_{30}$ value of about 0.5 mg/kg p.o. in tests measuring the inhibition of anaphylactic bronchospasm in sensitized guinea pigs having antigen-induced bronchoconstriction and an $ED_{50}$ value of about 4.5 mg/kg p.o. in tests measuring the reverse passive Arthus reaction in the pleural cavity of rats (as described by Myers et al., Inflammation, Vol. 9, No.1, 1985, pp. 91-98). Compound A has an $ED_{30}$ value of about 100 mg/kg in the splenic atrophy test as described above. These results for Compound A indicate that an immunosuppressive effective dose ($ED_{30}$) for such compounds is several times their anti-inflammatory and anti-allergy effective doses.

The usual dosage range for the compounds of formula I in a 70 kg mammal is an oral dose of about .1 to 250 mg/kg, preferably .1 to 150 mg/kg, in 3 or 4 divided doses per day. Of course, the dose will be regulated according to the potency of compound employed, the immunological disease being treated, and the judgment of the attending clinician depending on factors such as the degree and the severity of the disease state and age and general condition of the patient being treated.

To treat immunological diseases, the active compounds of formula I can be adminstered in unit dosage forms such as tablets, capsules, pills, powders, granules, sterile parenteral solutions or suspensions, suppositories, transermal compositions and the like. Such dosage forms are prepared according to standard techniques well known in the art.

In all of the above modes of treatment, the dosage to be administered and the route of administration depends upon the particular compound selected, the age and general health of the subject, and the severity and type of condition to be controlled. Thus, the dose ultimately provided must be left to the judgment of a trained health-care practitioner.

The quantity of active compound in a unit dose preparation may be varied or adjusted from 1 mg to 100 mg according to the particular application and the potency of the active ingredient and the intended treatment. The composition may, if desired, also contain other therapeutic agents.

When administered parenterally, e.g. intravenously, the compounds are administered at a dosage range of about 0.1 to 5 mg/kg of body weight in single or multiple daily doses.

The invention disclosed herein is exemplified by the following preparative examples. Alternative mechanistic pathways and analogous structures within the scope of applicants invention, may be apparent to those skilled in the art.

Preparative Example 1

1'-(3-Chlorophenyl)-1',4'-dihydro-4'-hydroxy-spiro[cyclopentane-1,3'(2'H)-[1,8]-naphthyridin]-2'-one

To a solution of 2.2 g (6.73 mmol.) of 1'-(3-chlorophenyl)-spiro[cyclopentane-1,3'-[1,8]-naphthyridin]-2',4'-(1'H)dione in 176 mL of (1:1) THF: EtOH (abs.) was added 2.54 g (40 mmol.) of $NaBH_3CN$ and 3.0 mL of acetic acid. The solution was stirred at room temperature. An additional 0.2 g (3.2 mmol.) of $NaBH_3CN$ and 0.3 mL of acetic acid were added on the fourth day. After 9 days the reaction was stopped by slow addition of 10 mL of water. After concentration, the residue was purified by column chromatography, eluting with $CHCl_3$-$CH_3OH$(95:5) to yield a colorless solid, 1.82 g. (5.53 mmol. 82%).

Recrystallization from $CHCl_3$ and diethyl ether ($Et_2O$) yielded the product, m.p. 144-146 °C.

Preparative Example 2

(SR,RS)-1-(3-Chlorophenyl)-4-hydroxy-1,3',4,4',5',6'-hexahydro-spiro[1,8-naphthyridine-3(2H),2'[2H]-pyran]-2-one (Compound A) and (RS,RS)-1-(3-Chlorophenyl)-4-hydroxy-1,3',4,4',5',6'-hexahydro-spiro[1,8,-naphthyridine-3(2H),2'[2H]-pyran]-2-one (Compound B)

To a solution of 1.0 g (2.92 mmol.) of 1-(4-chlorophenyl)-3',4',5',6'-tetrahydro-spiro[1,8-naphthyridine-3,2'-[2H]-pyran]-2,4-dione in 80 mL of (1:1) THF/$C_2H_5OH$ (abs.) was added 0.734 g (11.66 mmol.) of $NaBH_3CN$ and 0.6 mL of acetic acid. The solution was stirred at room temperature for 5 days, then it was quenched by the addition of 5 mL of water. After concentration, the residue was purified by column chromatography over silica gel, eluting with $CHCl_3$-$CH_3OH$ (95:5), to yield 1.06 g (95%) of a mixture of the diastereomers, Compounds A and B. These were separated by preparative reversed-phase HPLC, eluting with $CH_3CN$-$H_2O$-$CH_3COOH$ (30:70:1) to yield 0.52 g (54%) of Compound A, m.p. 183-184.5 °C, and 0.12 g (12%) of Compound B, m.p. 186.5-188.5 °C.

Preparative Example 3

4-Hydroxy-1,3',4,4',5',6'-hexahydro-1-phenyl-spiro[1,8-naphthyridine-3(2H),2'[2H]-pyran]-2-one (mixture of diastereomers)

In a stirred mixture of tetrahydrofuran (THF) (200 mL) and $C_2H_5OH$ (200 mL) was suspended 1-phenyl-3',4',5',6'-tetrahydro-spiro[1,8-naphthyridin-3,2'-[2H]-pyran]-2,4-dione (6.2 g) at room temperature. To the suspension was added acetic acid (2.6 mL) and tert-butylamine-borane (3.24 g).

After stirring for about 1 hour, water (ca. 200 mL) was added and the mixture was concentrated under vacuum to a volume of about 100 mL Additional water (400 mL) was added and the mixture was extracted with methylene chloride. The methylene chloride extract was separated, dried over sodium sulfate, filtered and concentrated. Ether was then added and the mixture was cooled. The resulting solid was removed by filtration, washed with fresh ether and dried to yield the desired product as a mixture of diastereomers, m.p. 211-213 °C.

By the methods described in Preparative Examples 1-3, the following compounds were prepared:
1'4'-Dihydro-4'-hydroxy-1'-phenyl-spiro[cyclopentane-1,3'-(2'H)[1,8]-naphthyridin]-2'-one, m.p. 148-150 °C; and 1',4'-dihydro-4'-hydroxy-1'-phenyl-spiro[furan-2,3-(2'H)[1,8]-naphythridin]-2'-one, mixture of diastereomers, m.p. 220-222 °C.

Example 1

7,8,9,10-Tetrahydro-5-phenyl-benzo[c][1,8]-naphthyridin-6-(5H)-one

A solution containing 1.2 g (4.07 mmol.) of 1',4'-dihydro-4'-hydroxy-1'-phenyl-spiro[cyclopentane-1,3'-(2'H)-[1,8]-naphthyridin]-2'-one in 5.0 mL of trifluoromethanesulfonic acid was stirred at room temperature for 1.75 hours.

To this solution was added 100 mL of water and the resulting solution was adjusted to pH 5.0 with 2N NaOH. The precipitate was collected by filtration, washed with water, and redissolved in 200 mL of $CH_2Cl_2$. The organic solution was washed twice with 50 mL of saturated $NaHCO_3$ solution, then with 100 mL of $H_2O$, dried with $MgSO_4$, filtered, and concentrated in vacuo to yield 0.62 g (55%) of material, which was recrystallized from $CHCl_3/Et_2O$ to yield the desired product, m.p. 160-162 °C.

Example 2

2,3-Dihydro-6-phenyl-1H-pyrano[2,3,-c][1,8]-naphthyridin-5(6H)-one,$\frac{1}{4}$ $H_2O$

To trifluoromethanesulfonic acid (5.0 mL) was added slowly, in portions, 1',4'-dihydro-4'-hydroxy-1'-phenylspiro[furan-2,3-(2'H)-[1,8]-naphythyridin]-2'-one (1.0g) using a solid powder addition funnel, under $N_2$ at room temperature. The reaction was followed by HPLC and TLC which showed that one diastereomer rearranged faster than the other but that both eventually disappeared, after a total of about 24 hours.

The resulting solution was poured into ice-water (200 mL) containing NaOH (2.26 g). The pH was then adjusted to 4 with 1 N HCl. The precipitate which formed on standing was collected, washed with water and purified by chromatography on silica gel (100 g), eluting with $CH_2Cl_2$ containing 2.5% of methanol, to yield the desired product, m.p. 250-252 °C.

In a similar manner, except for the length of time for which the reaction is run (progress followed by TLC), the following compounds were prepared:
5-(3-Chlorophenyl)-7,8,9,10-tetrahydro-benzo[c][1,8]-naphthyridin-6(5H)-one, m.p. 133-135 °C.;
7-Phenyl-1,2,3,4-tetrahydro-oxepino[2,3-c][1,8]-naphthyridin-6(7H)-one, m.p. 191-193 °C.; and
7-(3-Chlorophenyl)-1,2,3,4-tetrahydro-oxepino[2,3-c][1,8]-naphthyridin-6(7H)-one, m.p. 215-217 °C.

Example 3

2,3-Dihydro-6-phenyl-1H-pyrano[2,3-c] [1,8]-naphythyridin-5(6H)-thione.

A suspension of 2,3-dihydro-6-phenyl-1H-pyrano[2,3-c][1,8]-naphythyridin-5(6H)-one in dry toluene is stirred and heated in an $N_2$ atmosphere with a slight excess of Lawesson's Reagent [2,4-bis(4-methoxyphenyl)-1,3-dithia-2,4-diphosphetane-2,4-disulfide] at 50 °C then at gradually increasing temperatures (15 °C intervals, up to the reflux temperature) until TLC shows that reaction is occurring. Heating is

continued until essentially no starting material remains; then the mixture is cooled, filtered, evaporated and purified by chromatography to yield the desired product.

By following the procedure of Example 3, or modifications well known to one skilled in the art, other sulfur-containing analogs of the invention may be produced.

Example 4

1,2-Dihydro-5-phenylfuro[2,3-c][1,8] naphthyridin-4(5H)-one

Dissolve 3-n-butyl-4-hydroxy-1-phenyl-1,8-naphthyridin-2(1H)-one (10 g) in $CH_2Cl_2$ (1 L). Add a solution of $Br_2$ (1.74 mL, 5.43 g) in $CH_2Cl_2$ (30 mL) slowly. Stir briefly after the addition, then wash with water and evaporate. Recrystallize the intermediate bromo-compound (3-bromo-3-n-butyl-1-phenyl-1,8-naphthyridin-2,4(1H)-dione) from $CH_2Cl_2$/diethyl ether/hexane and redissolve it in $CH_2Cl_2$ (200 mL) and $CH_3OH$ (75 mL). Add DBU (12 mL, about 3 equiv.) and stir at room temperature. Evaporate off the solvents after about 1 hour and redissolve the residue in $CH_2Cl_2$ (750 mL). Add $H_2O$ and adjust the aqueous layer to pH of about 4 with 1N HCl. Dry with $MgSO_4$ and evaporate. Recrystalllze the product (3-n-butyl-3-methoxy-1-phenyl-1,8-naphthyridin-2,4(1H)-dione) from diethyl ether/$CH_2Cl_2$ and purify, if necessary, by chromatography over silica gel, eluting with $CH_2Cl_2$ containing 5% ethyl acetate.

Dissolve the purified product (5 g) in dry tetrahydrofuran (THF) (50 mL) and cool to below 0°C. Add a slight excess of vinyl magnesium bromide (1M in THF) and allow to react for 1 hour, then warm to room temperature. Add aqueous $NH_4Cl$ solution (50 mL), evaporate off the THF and extract into $CH_2Cl_2$ (2x50 mL). Wash with $H_2O$ (2x50 mL), dry with $Na_2SO_4$ and evaporate to yield a mixture of diastereomeric alcohols (diastereomers of 3-n-butyl-4-ethenyl-4-hydroxy-3-methoxy-1-phenyl-1,8-naphthyridin-2(1H)-one).

Dissolve the mixture in $CH_2Cl_2$ (50 mL) and cool to -78°5. Add a slight excess of $BBr_3$ in $CH_2Cl_2$ (10 mL) and stir for 1 hour. Allow to warm to room temperature, add $H_2O$ (25 mL) and adjust the pH of the aqueous layer to 4. Separate the organic layer, wash with water (2x25 mL), dry with $Na_2SO_4$, and evaporate. Dissolve the crude product (3-n-butyl-4-ethenyl-3,4-dihydroxy-1-phenyl-1,8-naphthyridin-2(1H)-one) in cold $CF_3SO_3H$ (below 0°C) (20 mL) and stir until reaction is complete, as shown by HPLC analysis of a small sample. Allow to warm to room temperature, then pour into ice-water, adjust the pH to 4 and extract with $CH_2Cl_2$ (2x50 mL). Wash the organic layer with $H_2O$ (2x25 mL), dry with $MgSO_4$, evaporate and purify by column chromatography over silica gel, eluting with increasing concentrations of ethyl acetate in $CH_2Cl_2$. Evaporate the fractions containing the product and recrystallise to yield the desired product.

Example 5

1,2-Dihydro-2-(hydroxymethyl)-4-phenyl-cyclobuta(c)[1,8]naphthyridin-3(4H)-one

A solution of 4-(2-propenyloxy)-1-phenyl-1,8-naphthyridin-2(1H)-one (1.0 g) in methanol (350 mL) under an atmosphere of nitrogen was irradiated at 3000Å for 3½ days. Solvent was removed under vacuum and the reaction product purified by chromatography on silica gel in $CH_2Cl_2$ containing 10% ethyl acetate. The fractions containing the product were combined and evaporated to yield 3(S R),9b(R S)-3,3a-dihydro-5-phenyl-3,9b-methano-2H-furo[3,2-c][1,8]naphthyridin-4(5H)-one, which was recrystallized from $CH_2Cl_2$/diethyl ether, m.p. 198-199.5°C.

This compound (1.5 g) was dissolved in $CH_3OH$ (100 mL). To the solution was added sodium methoxide (320 mg) and the mixture was heated at 90°C under a nitrogen atmosphere for about 2.5 hours. Solvent was removed under vacuum, the residue suspended in water, and the pH adjusted to 4 with 1N HCl. The product was extracted with $CH_2Cl_2$ (3x100 mL) and the combined extracts washed with water (100 mL), dried with $MgSO_4$, and partially evaporated before separation on a silica gel chromatography column. The column was first eluted with $CH_2Cl_2$ containing 10% ethyl acetate, followed by $CH_2Cl_2$ containing 5% $CH_3OH$, and the product isolated from the relevant fractions, m.p. 204-206°C.

Example 6

5-phenyl-8,9-dihydro-7H-cyclopenta[c][1,8]naphthyridin-6-(5H)-one 1/4 $H_2O$

6a To a stirred mixture of the product of Example 5, 1,2-dihydro-2-(hydroxymethyl)-4-phenyl-cyclobuta-(c)[1,8]naphthyridin-3(4H)-one and triethylamine (0.2 mL) in $CH_2Cl_2$ (5 mL) was added methanesulfonic anhydride (0.14g) at room temperature. After 2 hours at room temperature an additional quantity of

methanesulfonic anhydride (65 mg) was added. The reaction was followed by TLC (silica gel/$CH_2Cl_2$:5% MeOH). When the reaction was complete, the reaction mixture was evaporated to dryness, redissolved in fresh $CH_2Cl_2$, washed with 50 ml of $H_2O$ adjusted to a pH of about 8, dried ($MgSO_4$), and evaporated.

The crude mesylate (0.22g) was dissolved in $CF_3SO_3H$ (1 mL) at room temperature (in an $N_2$ atmosphere). After 2 days at room temperature the solution was heated at 80 °C for about 20 hours. After cooling, dilute NaOH solution was added and the mixture was adjusted to pH 4.5 with aqueous HCl solution. The product was extracted with $CH_2Cl_2$ and this extract was washed with $H_2O$, dried ($MgSO_4$), and evaporated to yield a mixture of the positonal double bond isomers.

6b The mixture of double bond positional isomers from 6a (50 mg) was dissolved in MeOH (10 mL) containing AcOH (0.1 mL). To the solution was added ammonium formate (36 mg) and 5% Pd/C (25 mg). The mixture was stirred (in an $N_2$ atmosphere) at room temperature and was followed by TLC (silica gel/$CH_2Cl_2$:5% MeOH). Additional quantities (x2) of ammonium formate (36 mg) were added after stirring had progressed for 0.5 hours and 4 hours. After 25 hours the solution was diluted with MeOH, filtered through Celite which was washed with more MeOH, and evaporated. The crude product was purified by chromatography (silica gel/$CH_2Cl_2$:5% MeOH) to yield the desired product, which was recrystallized from $Et_2O/CH_2Cl_2$, m.p. 230-232 °C; yield 75%.

By the methods of Examples 1-5 using suitably substituted reagents, the compounds according to Table III may be prepared.

TABLE III

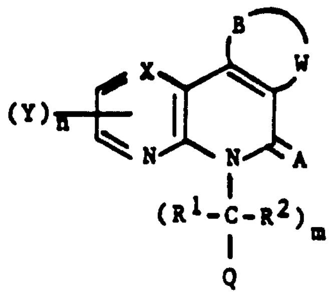

| X | n | Y* | m | $R^1$ | $R^2$ | A | B-W | Q |
|---|---|---|---|---|---|---|---|---|
| CH | 0 | - | 0 | - | - | O | $-CH_2CH_2CH_2O-$ | $C_6H_5-$ |
| CH | 0 | - | 0 | - | - | O | $-CH_2CH_2O-$ | $C_6H_5-$ |
| N | 0 | - | 0 | - | - | O | $-CH_2CH_2CH_2CH_2-$ | $C_6H_5-$ |
| N | 0 | - | 0 | - | - | O | $-CH_2CH_2CH_2CH_2-$ | $3-Cl-C_6H_4-$ |
| N | 0 | - | 0 | - | - | O | $-CH_2CH_2CH_2CH_2O-$ | $C_6H_5-$ |
| N | 0 | - | 0 | - | - | O | $-CH_2CH_2CH_2O-$ | $C_6H_5-$ |
| N | 0 | - | 0 | - | - | O | $-CH_2CH_2O-$ | $C_6H_5-$ |
| CH | 0 | - | 0 | - | - | S | $-CH_2CH_2CH_2CH_2O-$ | $C_6H_5-$ |
| CH | 0 | - | 0 | - | - | S | $-CH_2CH_2CH_2CH_2-$ | $C_6H_5-$ |
| CH | 1 | $7-CH_3$ | 0 | - | - | O | $-CH_2CH_2CH_2O-$ | $4-CH_3O-C_6H_4-$ |
| CH | 0 | - | 0 | - | - | O | $-CH_2CH(OH)CH_2O-$ | $C_6H_5-$ |
| CH | 0 | - | 0 | - | - | O | $-CH_2CH(CH_2OH)CH_2-$ | $C_6H_5-$ |
| CH | 0 | - | 0 | - | - | O | $-CH_2CH_2CH(CH_2OH)O-$ | $C_6H_5-$ |
| CH | 0 | - | 0 | - | - | O | $-CH_2CH_2CH_2CH_2O-$ | $3-CH_3S-C_6H_4-$ |
| CH | 0 | - | 0 | - | - | O | $-CH_2CH_2CH_2CH_2-$ | $3-NO_2-C_6H_4-$ |
| CH | 0 | - | 0 | - | - | O | $-CH_2CH_2CH_2-$ | $3-Br-C_6H_4-$ |
| CH | 0 | - | 1 | H | H | O | $-CH_2CH_2CH_2N(CH_3)-$ | $3-CF_3-C_6H_4-$ |
| CH | 0 | - | 1 | $CH_3$ | H | O | $-CH_2CH_2CH_2-$ | 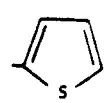 |
| CH | 1 | 6-Cl | 1 | H | H | O | $-CH_2CH_2CH_2-S^+(O-)-$ | $C_6H_5-$ |
| CH | 0 | - | 2 | H | H | O | $-CH_2CH_2CH_2O-$ | $4-F-C_6H_4-$ |
| CH | 0 | - | 1 | H | H | O | $-CH_2CH_2CH_2CH_2-$ | 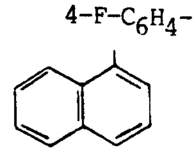 |
| CH | 0 | - | 1 | H | H | O | $-CH_2CH_2CH_2O-$ | 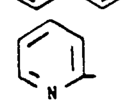 |

*Numbering based on 1,8-naphthyridine.

The following formulations exemplify some of the dosage forms of the compositions of this invention. In each, the term "active compound" designates 7-phenyl-1,2,3,4-tetrahydro-oxepino[2,3-c][1,8]-naphthyridin-6-(7H)-one. It is contemplated, however, that this compound may be replaced by equally effective amounts of other compounds of formula I.

Pharmaceutical Dosage Form Examples

Example A

Tablets

| No. | Ingredient | mg/tablet | mg/tablet |
|---|---|---|---|
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| | Total | 300 | 700 |

Method of Manufacture

Mix Item Nos. 1 and 2 in a suitable mixer for 10-15 minutes. Granulate the mixture with Item No. 3. Mill the damp granules through a coarse screen (e.g., 1/4") if needed. Dry the damp granules. Screen the dried granules if needed and mix with Item No. 4 and mix for 10-15 minutes. Add Item No. 5 and mix for 1-3 minutes. Compress the mixture to appropriate size and weight on a suitable tablet machine.

Example B

Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade | 40 | 70 |
| 4. | Magnesium Stearate NF | 4 | 7 |
| | Total | 250 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

Example C

Parenteral

| Ingredient | mg/vial | mg/vial |
|---|---|---|
| Active Compound Sterile Powder | 100 | 500 |

Add sterile water for injection or bacteriostatic water for injection, for reconstitution.

Example D

Injectable

| Ingredient | mg/vial |
|---|---|
| Active Compound | 100 |
| Methyl p-hydroxybenzoate | 1.8 |
| Propyl p-hydroxybenzoate | 0.2 |
| Sodium Bisulfite | 3.2 |
| Disodium Edetate | 0.1 |
| Sodium Sulfate | 2.6 |
| Water for Injection q.s. ad | 1.0 ml |

Method of Manufacture (for 1000 vials)

1. Dissolve p-hydroxybenzoate compounds in a portion (85% of the final volume) of the water for injection at 65-70°C.
2. Cool to 25-35°C. Charge and dissolve the sodium bisulfite, disodium edetate and sodium sulfate.
3. Charge and dissolve active compound.
4. Bring the solution to final volume by added water for injection.
5. Filter the solution through 0.22μ membrane and fill into appropriate containers.
6. Finally sterilize the units by autoclaving.

Example E

Nasal Spray

| | mg/ml |
|---|---|
| Active Compound | 10.0 |
| Phenyl Mercuric Acetate | 0.02 |
| Aminoacetic Acid USP | 3.7 |
| Sorbitol Solution, USP | 57.0 |
| Benzalkonium Chloride Solution | 0.2 |
| Sodium Hydroxide 1N Solution to adjust pH | |
| Water Purified USP to make | 1.0 ml |

The following formulations exemplify some of the dosage forms in which the anti-psoriatic agents of the invention may be employed.

Example F

Ointment

| Formula | mg/g |
|---|---|
| Active Compound | 1.0-20.0 |
| Benzyl Alcohol, NF | 20.0 |
| Mineral Oil, USP | 50.0 |
| White Petrolatum, USP to make | 1.0 g |

Method of Manufacture

Disperse active compound in a portion of the mineral oil. Mix and heat to 65°C, a weighed quantity of white petrolatum, the remaining mineral oil and benzyl alcohol, and cool to 50°-55°C. with stirring. Add the dispersed active compound to the above mixture with stirring. Cool to room temperature.

Example G

Cream

| Formula | mg/g |
|---|---|
| Active Compound | 1.0-20.0 |
| Stearic Acid, USP | 60.0 |
| Glyceryl Monostearate | 100.0 |
| Propylene Glycol, USP | 50.0 |
| Polyethylene Sorbitan Monopalmitate | 50.0 |
| Sorbitol Solution, USP | 30.0 |
| Benzyl Alcohol, NF | 10.0 |
| Purified Water, USP to make | 1.0 g |

Method of Manufacture

Heat the stearic acid, glyceryl monostearate and polyethylene sorbitan monopalmitate to 70°C. In a separate vessel, dissolve sorbital solution, benzyl alcohol, water, and half quantity of propylene glycol and heat to 70°C. Add the aqueous phase to oil phase with high speed stirring. Dissolve the active compound in remaining quantity of propylene glycol and add to the above emulsion when the temperature of emulsion is 37°-40°C. Mix uniformly with stirring and cool to room temperature.

Example H

Gel

| Formula | mg/g |
|---|---|
| Active Compound | 1.0-20.0 |
| Propylene Glycol, USP | 300.0 |
| Butylated Hydroxytoluene | 5.0 |
| Carbomer 940 | 5.0 |
| Sodium Hydroxide (added as a 1% w/w solution in propylene glycol) | 0.7 |
| Polyethylene Glycol 400, USP | 669.3-688. |

Method of Manufacture

Prepare a 1% solution of the sodium hydroxide in propylene glycol. Add approximately one-half the remaining propylene glycol and the polyethylene glycol 400 to a suitable vessel and mix. Dissolve the butylated hydroxytoluene in this mixture. Disperse Carbomer 940, a polymer of acrylic acid crosslinked with a polyfunctional agent, in the above mixture with vigorous agitation. Add the solution of sodium hydroxide with high speed agitation to bring pH up to 7 and mix until a thick gel forms. Dissolve the active compound in the remaining propylene glycol and add to the gel slowly as the gel is continuously mixed.

Example I

Lotion

| Formula | mg/g |
| --- | --- |
| Active Compound | 1.0-20.0 |
| Carbomer 940 | 3.0 |
| Sodium hydroxide (charged as 4% w/w aqueous solution) | 0.05 |
| Isopropyl Alcohol | 40.00 |
| Purified Water, USP to make | 1.0 g |

Method of Manufacture

Prepare a 4% solution of sodium hydroxide in water. Heat the purified water to 60 °C. Add Carbomer 940 and mix at high speed until dispersed. Cool the above mixture to room temperature and slowly charge sodium hydroxide solution until uniform. Add 80% of isopropyl alcohol to the above with mixing. Dissolve the active compound in remaining isopropyl alcohol. Add this to the mixture with stirring. Adjust pH to 5.0 to 5.5 with sodium hydroxide, if necessary.

Example J

Topical Aerosol

| Formula | mg/g |
| --- | --- |
| Active Compound | 1.0-20.0 |
| Caprylic/Capric Triglyceride | 50.00 |
| Mineral Oil | 20.00 |
| Denatured Alcohol | 150.00 |
| Hydrocarbon Aerosol Propellant q.s. ad. | 1.0 g |

Method of Manufacture

Add and mix the caprylic/capric triglyceride mineral oil and specially denatured alcohol in a suitable compounding tank. Add the active compound and continue mixing until the active compound is dissolved or dispersed uniformly. Fill the concentrate into cans and then fill the required amount of hydrocarbon aerosol propellant.

While the present invention has been described in conjunction with the specific embodiments set forth above, many alternatives, modifications and variations thereof will be apparent to those of ordinary skill in the art.

**Claims**

**Claims for the following Contracting States : BE, CH, DE, FR, GB, IT, LI, LU, NL, SE**

**1.** A compound having the structural formula I

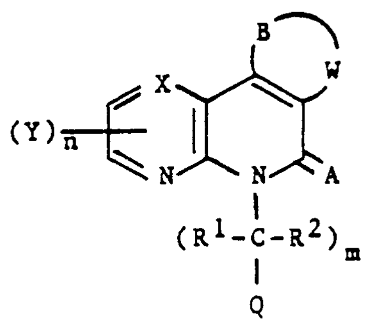

I

and pharmaceutically acceptable salts thereof, wherein:

X represents CH or N;

A represents O or S;

m is an integer of from 0 to 2;

n is an integer of from 0 to 2;

$R^1$ and $R^2$ are the same or different and each is independently selected from H or $C_{1-6}$ alkyl;

W represents a covalent bond or a group selected from -O-, $S(O)_p$-, -$N(R^4)$-, -NH-, -$N(COR^4)$-, and -$N(SO_2R^4)$- {wherein p is an integer of from O to 2 and $R^4$ is $C_{1-6}$ alkyl};

B represents alkylene having from 2 to 8 carbon atoms, which alkylene may be optionally substituted with a group selected from -OH, -F, alkyl having from 1 to 4 carbon atoms, -$CH_2OH$, -CHO, -$CO_2H$, -$COR^3$ {wherein $R^3$ is selected from -$NHR^4$, -$N(R^4)_2$ and -$OR^4$ and $R^4$ is as defined above}, and -CN, with the proviso that OH or F is not on the carbon adjacent to W when W is -O-, -$S(O)_p$-,-NH-, -$N(R^4)$-, -$N(COR^4)$ or -$N(SO_2R^4)$-; B also may represent an unsubstituted alkylene having one or more double bonds;

Q represents a $C_{6-15}$ aryl group or an aromatic heterocyclic group containing 3 to 14 carbon atoms and at least one O, S and/or N in the ring structure which can optionally be substituted with up to 3 substituents Y as defined below; and

each Y substituent is independently selected from -OH, hydroxymethyl, $C_{1-6}$ alkyl, halo, -$NO_2$, $C_{1-6}$ alkoxy, -$CF_3$, -CN, $C_{3-7}$ cycloalkyl, $C_{3-6}$ alkenyloxy, $C_{3-6}$ alkynyloxy, -$S(O)_p$-$R^4$ {wherein $R^4$ and p are as defined above}, -CO-$R^5$ {wherein $R^5$ represents -OH, -$NH_2$, -$NHR^4$, -$N(R^4)_2$ or -$OR^4$ in which $R^4$ is as defined above}, -O-D-$COR^5$ {wherein D represents alkylene having from 1 to 4 carbon atoms and $R^5$ is as defined above}, -$NH_2$, -$NHR^4$, -$N(R^4)_2$ {wherein $R^4$ is as defined above} and -NHCOH,

with the proviso that when

A = O and

B = alkylene having 4 carbon atoms and

W = a covalent bond and

Q = unsubstituted $C_{6-15}$ aryl

then X represents N.

2. The compound of claim 1 having formula

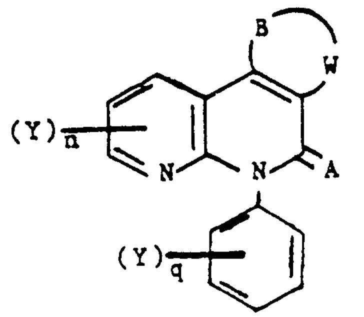

II

wherein A, B, W, Y and n are as defined in claim 1 and q is 0 to 2, and wherein, if n + q is greater than 1, the Y groups may be the same or different.

3. The compound of either claims 1 or 2 wherein:
-B-W- represents an alkylene group or an alkyleneoxy group either of which may be substituted;

4. The compound of any of claims 1-3 above wherein
B-W represents $-(CH_2)_4-$, $-(CH_2)_5-$, $(CH_2)_4-O-$ or $(CH_2)_3-O$; and,
n is zero.

5. The compound of any of claims 1-4 above wherein:
A is oxygen and the Y substituents on the phenyl ring are each independently chloro, nitro or trifluoromethyl.

6. A compound according to claim 1 selected from
5-phenyl-7,8,9,10-tetrahydro-benzo[c][1,8]-naphthyridin-6(5H)-one;
5-(3-chlorophenyl)-7,8,9,10-tetrahydro-benzo[c][1,8]-naphthyridin-6(5H)-one;
7-phenyl-1,2,3,4-tetrahydro-oxepino[2,3-c][1,8]-naphthyridin-6(7H)-one;
7-(3-chlorophenyl)-1,2,3,4-tetrahydro-oxepino[2,3-c][1,8]-naphthyridin-6(7H)-one;
2,3-dihydro-6-phenyl-1H-pyrano[2,3-c][1,8]-naphthyridin-5(6H)-one.
1,2-dihydro-5-phenylfuro[2,3-c][1,8]-naphthyridin-4[5H]-one; or
1,2-dihydro-2-(hydroxymethyl)-4-phenylcyclobuta[c][1,8]naphthyridin-3(4H)-one.
5-phenyl-8,9,dihydro-7H-cyclopenta [c][1,8]naphthyridin-6(5H)-one 1/4 $H_2O$.

7. A pharmaceutical composition which comprises a compound having the structure formula I

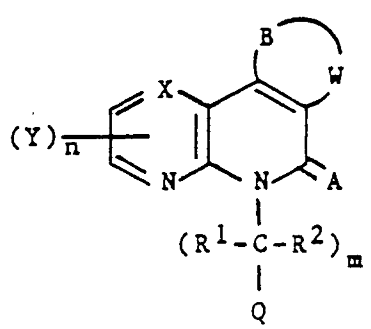

I

and pharmaceutically acceptable salts thereof, wherein:
X represents CH or N;
A represents O or S;
m is an integer of from 0 to 2;
n is an integer of from 0 to 2;
$R^1$ and $R^2$ are the same or different and each is independently selected from H or $C_{1-6}$ alkyl;

W represents a covalent bond or a group selected from -O-, $-S(O)_p-$, $-N(R^4)-$, -NH-, $-N(COR^4)-$, and $-N(SO_2R^4)-$ {wherein p is an integer of from O to 2 and $R^4$ is $C_{1-6}$ alkyl};

B represents alkylene having from 2 to 8 carbon atoms, which alkylene may be optionally substituted with a group selected from -OH, -F, alkyl having from 1 to 4 carbon atoms, $-CH_2OH$, -CHO, $-CO_2H$, $-COR^3$ {wherein $R^3$ is selected from $-NHR^4$, $-N(R^4)_2$ and $-OR^4$ and $R^4$ is as defined above}, and -CN, with the proviso that OH or F is not on the carbon adjacent to W when W is -O-, $-S(O)_p-$,-NH-, $-N(R^4)-$, $-N(COR^4)$ or $-N(SO_2R^4)-$; B also may represent an unsubstituted alkylene having one or more double bonds;

Q represents a $C_{6-15}$ aryl group or an aromatic heterocyclic group containing 3 to 14 carbon atoms and at least one O, S and/or N in the ring structure which can optionally be substituted with up to 3 substituents Y as defined below; and

each Y substituent is independently selected from -OH, hydroxymethyl, $C_{1-6}$ alkyl, halo, $-NO_2$, $C_{1-6}$ alkoxy, $-CF_3$, -CN, $C_{3-7}$ cycloalkyl, $C_{3-6}$ alkenyloxy, $C_{3-6}$ alkynyloxy, $-S(O)_p-R^4$ {wherein $R^4$ and p are as defined above}, $-CO-R^5$ {wherein $R^5$ represents -OH, $-NH_2$, $-NHR^4$, $-N(R^4)_2$ or $-OR^4$ in which $R^4$ is as defined above}, $-O-D-COR^5$ {wherein D represents alkylene having from 1 to 4 carbon atoms and $R^5$ is as defined above}, $-NH_2$, $-NHR^4$, $-N(R^4)_2$ {wherein $R^4$ is as defined above} and -NHCOH, in combination with a pharmaceutically acceptable carrier.

**8.** A composition according to Claim 7 wherein said compound has the formula

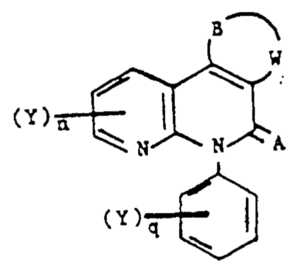

II

wherein A, B, W, Y and n are as defined in claim 1 and q is 0 to 2, and wherein, if n+g is greater than 1, the Y groups may be the same or different.

**9.** A composition according to Claim 7 or Claim 8 wherein -B-W- represents an alkylene group or an alkyleneoxy group either of which may be substituted;

**10.** A composition according to any of Claims 7 to 9 wherein B-W represents $-(CH_2)_4-$, $-(CH_2)_5-$, $(CH_2)_4-O-$ or $(CH_2)_3-O$; and, n is zero.

**11.** A composition according to any of Claims 7 to 10 wherein A is oxygen and the Y substitutents on the phenyl ring are each independently chloro, nitro or trifluoromethyl.

**12.** A composition according to Claim 7 wherein said compound is selected from

5-phenyl-7,8,9,10-tetrahydro-benzo[c][1,8]-naphthyridin-6(5H)-one;
5-(3-chlorophenyl)-7,8,9,10-tetrahydro-benzo[c][1,8]-naphthyridin-6(5H)-one;
7-phenyl-1,2,3,4-tetrahydro-oxepino[2,3-c][1,8]-naphthyridin-6(7H)-one;
7-(3-chlorophenyl)-1,2,3,4-tetrahydro-oxepino[2,3-c][1,8]-naphthyridin-6(7H)-one;
2,3-dihydro-6-phenyl-1H-pyrano[2,3-c][1,8]-naphthyridin-5(6H)-one.
1,2-dihydro-5-phenylfuro[2,3-c][1,8]-naphthyridin-4[5H]-one; or
1,2-dihydro-2-(hydroxymethyl)-4-phenylcyclobuta[c][1,8]naphthyridin-3(4H)one.
5-phenyl-8,9,dihydro-7H-cyclopenta [c][1,8]naphthyridin-6(5H)-one 1/4 $H_2O$.

**13.** The use of a compound of formula I defined in any of Claims 7 to 12 in the manufacture of a pharmaceutical composition for treating allergic reactions in a mammal.

**14.** The use of a compound of formula I defined in any of Claims 7 to 12 in the manufacture of a pharmaceutical composition for treating inflammation in a mammal.

15. The use of a compound of formula I defined in any of Claims 7 to 12 in the manufacture of a pharmaceutical composition for treating peptic ulcers in a mammal.

16. The use of a compound of formula I defined in any of Claims 7 to 12 in the manufacture of a pharmaceutical composition for treating hyperproliferative skin disease in a mammal.

17. The use of a compound of formula I defined in any of Claims 7 to 12 in the manufacture of a pharmaceutical composition for suppressing the immune response in a mammal.

18. A process for preparing a compound of the formula I

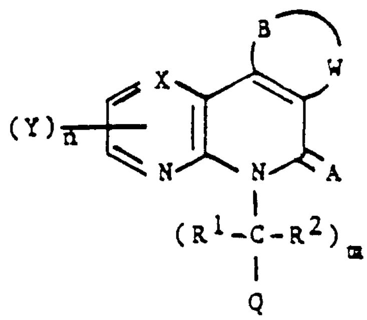

I

said process characterized by A.

a) treating the compound of formula IV

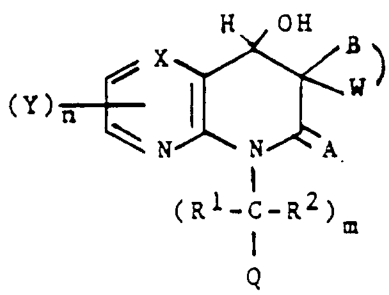

IV

with a super acid having a Hammett acidity function of less than minus 12 to form a compound of formula I,

b) contacting a compound of the formula

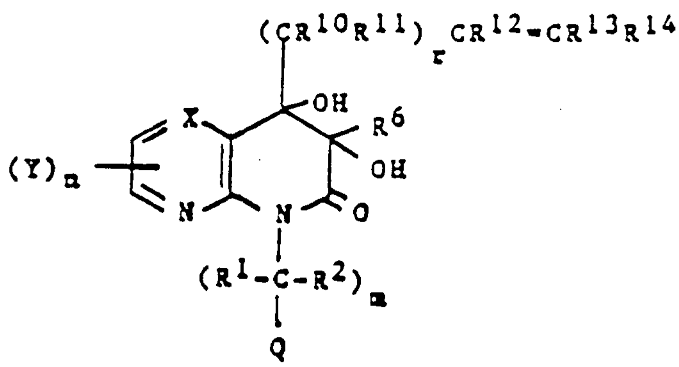

XI.

with a strong organic or inorganic acid to produce compound I;

c) contacting a compound of the formula

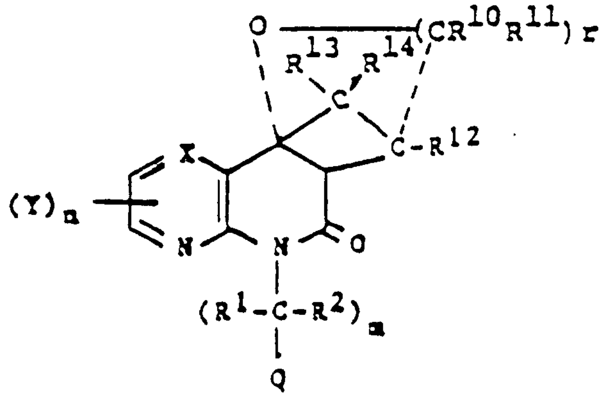

with a strong base to form a compound of formula I; or

d) contacting a compound of the formula

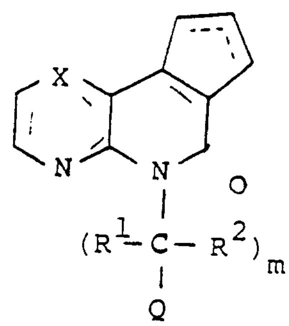

with a reducing agent to produce a compound of the formula

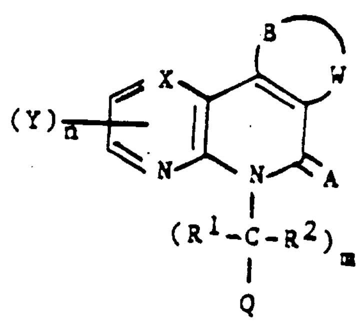

I

wherein X, A, m, n, W, B, Q, Y, $R^1$, $R^2$ are as defined in claim 1 and wherein

$R^6$ is an alkyl group having from 2 to 8 carbon atoms;

$R^{10}$, $R^{11}$, $R^{12}$, $R^{13}$, and $R^{14}$ independently are H or an alkyl having from 1 to 4 carbon atoms; and

r is 0 or 1.

**Claims for the following Contracting States : AT, ES, GR**

**1.** A process for producing a compound having the structural formula I

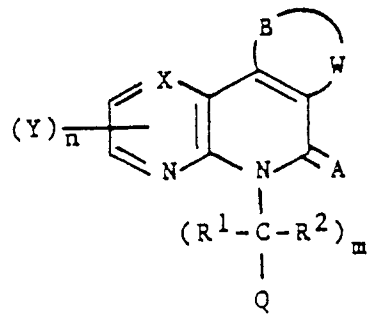

I

and pharmaceutically acceptable salts thereof, wherein:

X represents CH or N;

A represents O or S;

m is an integer of from 0 to 2;

n is an integer of from 0 to 2;

$R^1$ and $R^2$ are the same or different and each is independently selected from H or $C_{1-6}$ alkyl;

W represents a covalent bond or a group selected from -O-, $-S(O)_p-$, $-N(R^4)-$, -NH-, $-N(COR^4)-$, and $-N(SO_2R^4)-$ {wherein p is an integer of from O to 2 and $R^4$ is $C_{1-6}$ alkyl};

B represents alkylene having from 2 to 8 carbon atoms, which alkylene may be optionally substituted with a group selected from -OH, -F, alkyl having from 1 to 4 carbon atoms, $-CH_2OH$, -CHO, $-CO_2H$, $-COR^3$ {wherein $R^3$ is selected from $-NHR^4$, $-N(R^4)_2$ and $-OR^4$ and $R^4$ is as defined above}, and -CN, with the proviso that OH or F is not on the carbon adjacent to W when W is -O-, $-S(O)_p-$,-NH-, $-N(R^4)-$, $-N(COR^4)$ or $-N(SO_2R^4)-$; B also may represent an unsubstituted alkylene having one or more double bonds;

Q represents a $C_{6-15}$ aryl group or an aromatic heterocyclic group containing 3 to 14 carbon atoms and at least one O, S and/or N in the ring structure which can optionally be substituted with up to 3 substituents Y as defined below; and

each Y substituent is independently selected from -OH, hydroxymethyl, $C_{1-6}$ alkyl, halo, $-NO_2$, $C_{1-6}$ alkoxy, $-CF_3$, -CN, $C_{3-7}$ cycloalkyl, $C_{3-6}$ alkenyloxy, $C_{3-6}$ alkynyloxy, $-S(O)_p-R^4$ {wherein $R^4$ and p are as defined above}, $-CO-R^5$ {wherein $R^5$ represents -OH, $-NH_2$, $-NHR^4$, $-N(R^4)_2$ or $-OR^4$ in which $R^4$ is as defined above}, $-O-D-COR^5$ {wherein D represents alkylene having from 1 to 4 carbon atoms and $R^5$ is as defined above}, $-NH_2$, $-NHR^4$, $-N(R^4)_2$ {wherein $R^4$ is as defined above} and -NHCOH,

with the proviso that when

A = O and

B = alkylene having 4 carbon atoms and

W = a covalent bond and

Q = unsubstituted $C_{6-15}$ aryl

then X represents N, said process characterised by

(a) treating the compound of formula IV

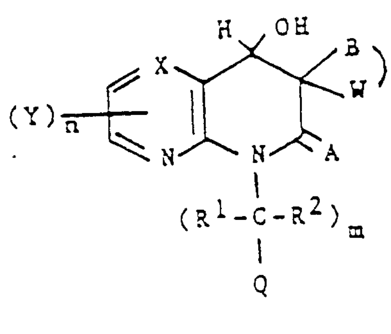

IV

with a super acid having a Hammett acidity function of less than minus 12 to form a compound of

formula I,
b) contacting a compound of the formula

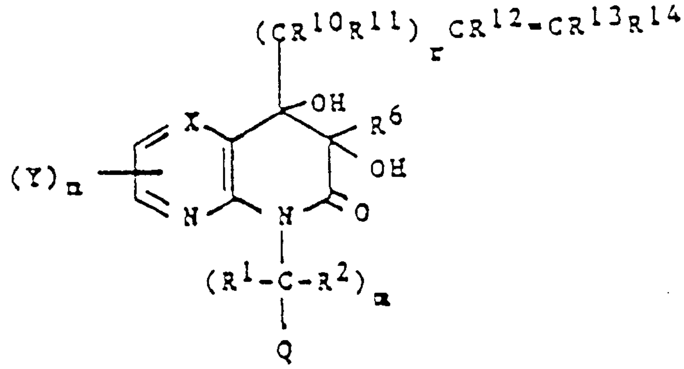

XI.

with a strong organic or inorganic acid to produce compound I;
c) contacting a compound of the formula

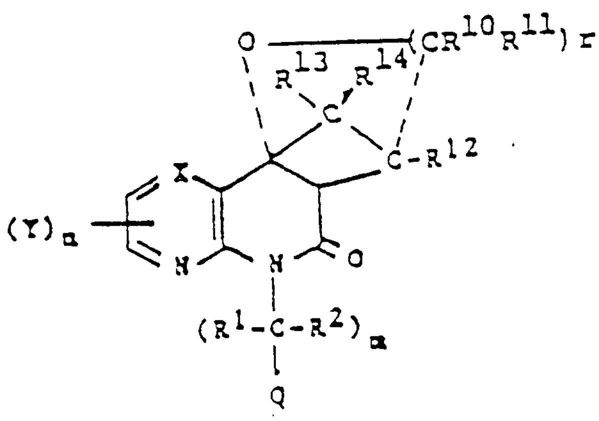

with a strong base to form a compound of formula I; or
d) contacting a compound of the formula

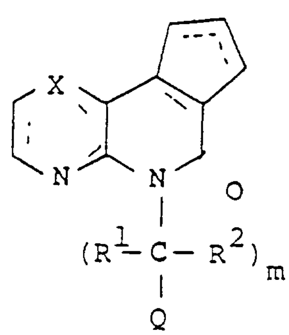

with a reducing agent to produce a compound of the formula

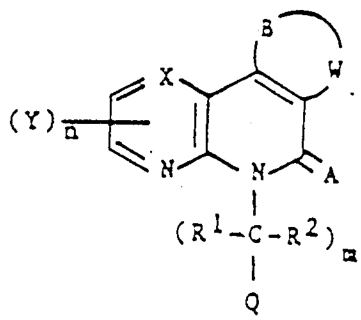

I

wherein X, A, m, n, W, B, Q, Y, $R^1$, $R^2$ are as previously defined and wherein

$R^6$ is an alkyl group having from 2 to 8 carbon atoms;

$R^{10}$, $R^{11}$, $R^{12}$, $R^{13}$, and $R^{14}$ independently are H or an alkyl having from 1 to 4 carbon atoms; and

r is 0 or 1.

**2.** The process of claim 1 wherein said produced compound has the formula

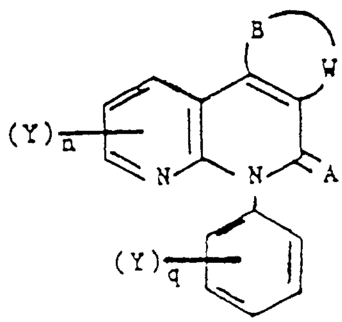

II

wherein A, B, W, Y and n are as defined in claim 1 and q is 0 to 2, and wherein, if n + q is greater than 1, the Y groups may be the same or different.

**3.** The process of either claims 1 or 2 wherein:

-B-W- represents an alkylene group or an alkyleneoxy group either of which may be substituted;

**4.** The process of any of claims 1-3 above wherein

B-W represents $-(CH_2)_4-$, $-(CH_2)_5-$, $(CH_2)_4-O-$ or $(CH_2)_3-O$; and,

n is zero.

**5.** The process of any of claims 1-4 above wherein:

A is oxygen and the Y substituents on the phenyl ring are each independently chloro, nitro or trifluoromethyl.

**6.** A process according to claim 1 wherein said produced compound is selected from

5-pheny1-7,8,9,10-tetrahydro-benzo[c][1,8]-naphthyridin-6(5H)-one;

5-(3-chlorophenyl)-7,8,9,10-tetrahydro-benzo[c][1,8]-naphthyridin-6(5H)-one;

7-phenyl-1,2,3,4-tetrahydro-oxepino[2,3-c][1,8]-naphthyridin-6(7H)-one;

7-(3-chlorophenyl)-1,2,3,4-tetrahydro-oxepino[2,3-c][1,8]-naphthyridin-6(7H)-one;

2,3-dihydro-6-phenyl-1H-pyrano[2,3-c][1,8]-naphthyridin-5(6H)-one.

1,2-dihydro-5-phenylfuro[2,3-c][1,8]-naphthyridin-4[5H]-one; or

1,2-dihydro-2-(hydroxymethyl)-4-phenylcyclobuta[c][1,8]naphthyridin-3(4H)-one.

5-phenyl-8,9,dihydro-7H-cyclopenta [c][1,8]naphthyridin-6(5H)-one 1/4 $H_2O$.

**7.** A method for producing a pharmaceutical composition which comprises admixing a compound having the structural formula

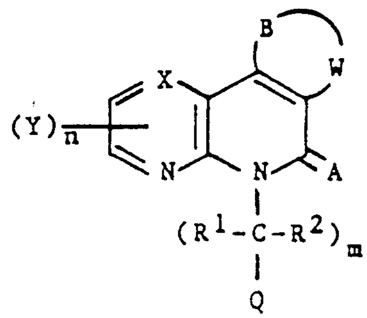

I

and pharmaceutically acceptable salts thereof, wherein:

X represents CH or N;

A represents O or S;

m is an integer of from 0 to 2;

n is an integer of from 0 to 2;

$R^1$ and $R^2$ are the same or different and each is independently selected from H or $C_{1-6}$ alkyl;

W represents a covalent bond or a group selected from -O-, $-S(O)_p-$, $-N(R^4)-$, -NH-, $-N(COR^4)-$, and $-N(SO_2R^4)-$ {wherein p is an integer of from O to 2 and $R^4$ is $C_{1-6}$ alkyl};

B represents alkylene having from 2 to 8 carbon atoms, which alkylene may be optionally substituted with a group selected from -OH, -F, alkyl having from 1 to 4 carbon atoms, $-CH_2OH$, -CHO, $-CO_2H$, $-COR^3$ {wherein $R^3$ is selected from $-NHR^4$, $-N(R^4)_2$ and $-OR^4$ and $R^4$ is as defined above}, and -CN, with the proviso that OH or F is not on the carbon adjacent to W when W is -O-, $-S(O)_p-$,-NH-, $-N(R^4)-$, $-N(COR^4)$ or $-N(SO_2R^4)-$; B also may represent an unsubstituted alkylene having one or more double bonds;

Q represents a $C_{6-15}$ aryl group or an aromatic heterocyclic group containing 3 to 14 carbon atoms and at least one O, S and/or N in the ring structure which can optionally be substituted with up to 3 substituents Y as defined below; and

each Y substituent is independently selected from -OH, hydroxymethyl, $C_{1-6}$ alkyl, halo, $-NO_2$, $C_{1-6}$ alkoxy, $-CF_3$, -CN, $C_{3-7}$ cycloalkyl $C_{3-6}$ alkenyloxy, $C_{3-6}$ alkynyloxy, $-S(O)_p-R^4$ {wherein $R^4$ and p are as defined above}, $-CO-R^5$ {wherein $R^5$ represents -OH, $-NH_2$, $-NHR^4$, $-N(R^4)_2$ or $-OR^4$ in which $R^4$ is as defined above}, $-O-D-COR^5$ {wherein D represents alkylene having from 1 to 4 carbon atoms and $R^5$ is as defined above}, $-NH_2$, $-NHR^4$, $-N(R^4)_2$ {wherein $R^4$ is as defined above} and -NHCOH, with a pharmaceutically acceptable carrier.

**8.** A method according to Claim 7 wherein said compound has the formula

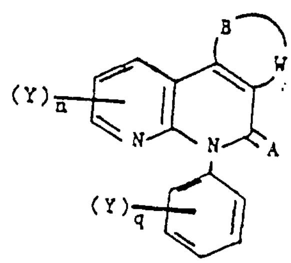

wherein A, B, W, Y and n are as defined in claim 1 and q is 0 to 2, and wherein, if n + g is greater than 1, the Y groups may be the same or different.

**9.** A method according to Claim 7 or Claim 8 wherein -B-W- represents an alkylene group or an alkyleneoxy group either of which may be substituted;

**10.** A method according to any of Claims 7 to 9 wherein B-W represents $-(CH_2)_4-$, $-(CH_2)_5-$, $(CH_2)_4-O-$ or $(CH_2)_3-O$; and, n is zero.

11. A method according to any of Claims 7 to 10 wherein A is oxygen and the Y substitutents on the phenyl ring are each independently chloro, nitro or trifluoromethyl.

12. A method according to Claim 7 wherein said compound is selected from
5-phenyl-7,8,9,10-tetrahydro-benzo[c][1,8]-naphthyridin-6(5H)-one;
5-(3-chlorophenyl)-7,8,9,10-tetrahydro-benzo[c][1,8]-naphthyridin-6(5H)-one;
7-phenyl-1,2,3,4-tetrahydro-oxepino[2,3-c][1,8]-naphthyridin-6(7H)-one;
7-(3-chlorophenyl)-1,2,3,4-tetrahydro-oxepino[2,3-c][1,8]-naphthyridin-6(7H)-one;
2,3-dihydro-6-phenyl-1H-pyrano[2,3-c][1,8]-naphthyridin-5(6H)-one.
1,2-dihydro-5-phenylfuro[2,3-c][1,8]-naphthyridin-4[5H]-one; or
1,2-dihydro-2-(hydroxymethyl)-4-phenylcyclobuta[c][1,8]naphthyridin-3(4H)-one.
5-phenyl-8,9,dihydro-7H-cyclopenta [c][1,8]naphthyridin-6(5H)-one 1/4 $H_2O$.

13. The use of a compound of formula I defined in any of Claims 7 to 12 in the manufacture of a pharmaceutical composition for treating allergic reactions in a mammal.

14. The use of a compound of formula I defined in any of Claims 7 to 12 in the manufacture of a pharmaceutical composition for treating inflammation in a mammal.

15. The use of a compound of formula I defined in any of Claims 7 to 12 in the manufacture of a pharmaceutical composition for treating peptic ulcers in a mammal.

16. The use of a compound of formula I defined in any of Claims 7 to 12 in the manufacture of a pharmaceutical composition for treating hyperproliferative skin disease in a mammal.

17. The use of a compound of formula I defined in any of Claims 7 to 12 in the manufacture of a pharmaceutical composition for suppressing the immune response in a mammal.

**Patentansprüche**

**Patentansprüche für folgende Vertragsstaaten : BE, CH, DE, FR, GB, IT, LI, LU, NL, SE**

1. Verbindung mit der Strukturformel I

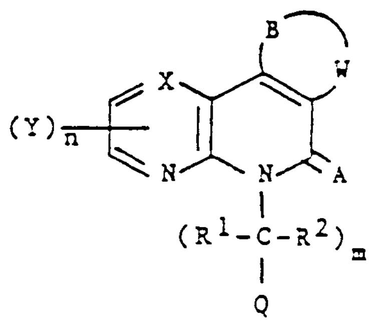

I

und deren pharmazeutisch annehmbare Salze, worin:

| | |
|---|---|
| X | CH oder N bedeutet; |
| A | O oder S bedeutet; |
| m | eine ganze Zahl von 0 bis 2 ist; |
| n | eine ganze Zahl von 0 bis 2 ist; |
| $R^1$ und $R^2$ | gleich oder verschieden sind und jeweils unabhängig ausgewählt sind aus H oder $C_{1-6}$-Alkyl; |
| W | eine covalente Bindung bedeutet oder eine Gruppe, die ausgewählt ist aus -O-, $-S(O)_p-$, $-N(R^4)-$, -NH-, $-N(COR^4)-$ und $-N(SO_2R^4)-$ (worin p eine ganze Zahl von 0 bis 2 und $R^4$ $C_{1-6}$-Alkyl ist); |
| B | Alkylen mit 2 bis 8 Kohlenstoff-Atomen bedeutet, wobei das Alkylen gegebenenfalls mit einer Gruppe substituiert sein kann, ausgewählt aus -OH, -F, Alkyl mit 1 bis 4 Kohlenstoff-Atomen, $-CH_2OH$, -CHO, $-CO_2H$, $-COR^3$ {worin $R^3$ ausgewählt ist aus |

-$NHR^4$, -$N(R^4)_2$ und -$OR^4$, und $R^4$ wie oben definiert ist} und -CN, mit der Maßgabe, daß OH oder F sich nicht an dem W benachbarten Kohlenstoff-Atom befindet, wenn W -O-, $S(O)_p$-, -NH-, -$N(R^4)$-, -$N(COR^4)$- oder -$N(SO_2R^4)$- ist, wobei B auch ein unsubstituiertes Alkylen mit einer oder mehreren Doppelbindungen sein kann;

Q eine $C_{6-15}$-Aryl-Gruppe bedeutet oder eine aromatische heterocyclische Gruppe, die 3 bis 14 Kohlenstoff-Atome und wenigstens ein O, S und/oder N in der Ringstruktur enthält und gegebenenfalls substituiert sein kann mit bis zu drei Substituenten Y wie nachstehend definiert; und jeder Substituent Y unabhängig ausgewählt ist aus -OH, Hydroxymethyl, $C_{1-6}$-Alkyl, Halogen, -$NO_2$, $C_{1-6}$-Alkoxy, -$CF_3$, -CN, $C_{3-7}$-Cycloalkyl, $C_{3-6}$-Alkenyloxy, $C_{3-6}$-Alkinyloxy, -$S(O)_p$-$R^4$ (worin $R^4$ und p wie vorstehend definiert sind), -CO-$R^5$ {worin $R^5$ -OH, -$NH_2$, -$NHR^4$, -$N(R^4)_2$ oder -$OR^4$ bedeutet, worin $R^4$ wie oben definiert ist}, -O-D-$COR^5$ {worin D Alkylen mit 1 bis 4 Kohlenstoff-Atomen bedeutet und $R^5$ wie oben definiert ist}, -$NH_2$, -$NHR^4$, -$N(R^4)_2$ (worin $R^4$ wie oben definiert ist) und NHCOH,

mit der Maßgabe, daß wenn

A = O und
B = Alkylen mit 4 Kohlenstoff-Atomen und
W = eine covalente Bindung und
Q = unsubstituiertes $C_{6-15}$-Aryl,
X dann N bedeutet.

2. Verbindung nach Anspruch 1 mit der Formel

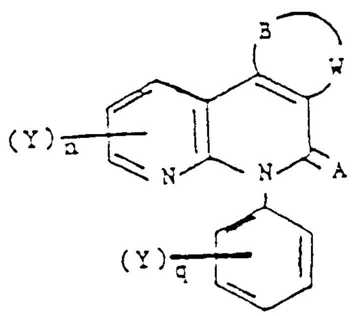

II

worin A, B, W, Y und n wie in Anspruch 1 definiert sind, und q 0 bis 2 ist, und worin, falls n + q größer als 1 ist, die Y-Gruppen gleich oder verschieden sein können.

3. Verbindung nach Anspruch 1 oder 2, worin -B-W- eine Alkylen-Gruppe oder eine Alkylenoxy-Gruppe bedeutet, die beide substituiert sein können.

4. Verbindung nach irgendeinem der vorstehenden Ansprüche 1 bis 3, worin B-W -$(CH_2)_4$-, -$(CH_2)_5$-, -$(CH_2)_4$-O- oder -$(CH_2)_3$-O- bedeutet, und n null ist.

5. Verbindung nach irgendeinem der vorstehenden Ansprüche 1 bis 4, worin A Sauerstoff ist, und die Y-Substituenten am Phenyl-Ring jeweils unabhängig Chlor, Nitro oder Trifluormethyl sind.

6. Verbindung nach Anspruch 1, ausgewählt aus 5-Phenyl-7,8,9,10-tetrahydro-benzo[c][1,8]naphthyridin-6-(5H)-on;
5-(3-Chlorphenyl)-7,8,9,10-tetrahydrobenzo[c][1,8]naphthyridin-6(5H)-on;
7-Phenyl-1,2,3,4-tetrahydro-oxepino[2,3-c][1,8]naphthyridin-6(7H)-on;
7-(3-Chlorphenyl)-1,2,3,4-tetrahydro-oxepino[2,3-c][1,8]naphthyridin-6(7H)-on;
2,3-Dihydro-6-phenyl-1H-pyrano[2,3-c][1,8]naphthyridin-5(6H)-on;
1,2-Dihydro-5-phenylfuro[2,3-c][1,8]naphthyridin-4(5H)-on; oder
1,2-Dihydro-2-(hydroxymethyl)-4-phenylcyclobuta[c][1,8]naphthyridin-3(4H)-on,
5-Phenyl-8,9-dihydro-7H-cyclopenta[c][1,8]naphthyridin-6(5H)-on • 1/4 $H_2O$.

**7.** Pharmazeutische Zusammensetzung, umfassend eine Verbindung mit der Strukturformel I

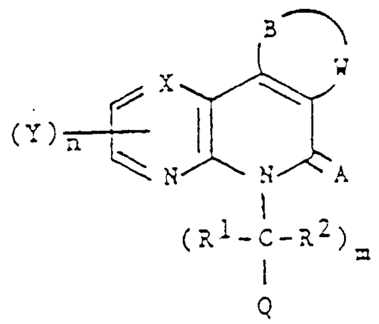

I

und pharmazeutisch annehmbare Salze derselben, worin:

| | |
|---|---|
| X | CH oder N bedeutet; |
| A | O oder S bedeutet; |
| m | eine ganze Zahl von 0 bis 2 ist; |
| n | eine ganze Zahl von 0 bis 2 ist; |
| $R^1$ und $R^2$ | gleich oder verschieden sind und jeweils unabhängig ausgewählt sind aus H oder $C_{1-6}$-Alkyl; |
| W | eine covalente Bindung bedeutet oder eine Gruppe, die ausgewählt ist aus -O-, $S(O)_p$-, -$N(R^4)$-, -NH-, -$N(COR^4)$- und -$N(SO_2R^4)$- (worin p eine ganze Zahl von 0 bis 2 und $R^4$ $C_{1-6}$-Alkyl ist); |
| B | Alkylen mit 2 bis 8 Kohlenstoff-Atomen bedeutet, wobei das Alkylen gegebenenfalls mit einer Gruppe substituiert sein kann, ausgewählt aus -OH, -F, Alkyl mit 1 bis 4 Kohlenstoff-Atomen, -$CH_2OH$, -CHO, -$CO_2H$, -$COR^3$ {worin $R^3$ ausgewählt ist aus -$NHR^4$, -$N(R^4)_2$ und -$OR^4$, und $R^4$ wie oben definiert ist} und -CN, mit der Maßgabe, daß OH oder F sich nicht an dem W benachbarten Kohlenstoff-Atom befindet, wenn W -O-, -$S(O)_p$-, -NH-, -$N(R^4)$-, -$N(COR^4)$- oder -$N(SO_2R^4)$- ist, wobei B auch ein unsubstituiertes Alkylen mit einer oder mehreren Doppelbindungen sein kann; |
| Q | eine $C_{6-15}$-Aryl-Gruppe bedeutet oder eine aromatische heterocyclische Gruppe, die 3 bis 14 Kohlenstoff-Atome und wenigstens ein O, S und/oder N in der Ringstruktur enthält und gegebenenfalls substituiert sein kann mit bis zu drei Substituenten Y wie nachstehend definiert; und jeder Substituent Y unabhängig ausgewählt ist aus -OH, Hydroxymethyl, $C_{1-6}$-Alkyl, Halogen, -$NO_2$, $C_{1-6}$-Alkoxy, -$CF_3$, -CN, $C_{3-7}$-Cycloalkyl, $C_{3-6}$-Alkenyloxy, $C_{3-6}$-Alkinyloxy, -$S(O)_p$-$R^4$ (worin $R^4$ und p wie vorstehend definiert sind), -CO-$R^5$ {worin $R^5$ -OH, -$NH_2$, -$NHR^4$, -$N(R^4)_2$ oder -$OR^4$ bedeutet, worin $R^4$ wie oben definiert ist}, -O-D-$COR^5$ {worin D Alkylen mit 1 bis 4 Kohlenstoff-Atomen bedeutet und $R^5$ wie oben definiert ist}, -$NH_2$, -$NHR^4$, -$N(R^4)_2$ (worin $R^4$ wie oben definiert ist) und NHCOH, |

in Kombination mit einem pharmazeutisch annehmbaren Träger.

**8.** Zusammensetzung nach Anspruch 7, wobei die Verbindung die Formel

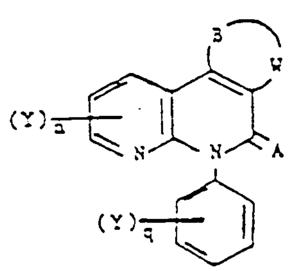

II

aufweist, worin A, B, W, Y und n wie in Anspruch 1 definiert sind, und q 0 bis 2 ist, und worin, falls n + g größer ist als 1, die Y-Gruppen gleich oder verschieden sein können.

**9.** Zusammensetzung nach Anspruch 7 oder 8, worin -B-W- eine Alkylen-Gruppe oder eine Alkylenoxy-Gruppe bedeutet, die beide substituiert sein können.

**10.** Zusammensetzung nach irgendeinem der Ansprüche 7 bis 9, worin B-W $-(CH_2)_4-$, $-(CH_2)_5-$, $-(CH_2)_4-O-$ oder $-(CH_2)_3-O-$ bedeutet, und n null ist.

**11.** Zusammensetzung nach irgendeinem der Ansprüche 7 bis 10, worin A Sauerstoff ist und die Y-Substituenten am Phenyl-Ring jeweils unabhängig Chlor, Nitro oder Trifluormethyl sind.

**12.** Zusammensetzung nach Anspruch 7, wobei die Verbindung ausgewählt ist aus
5-Phenyl-7,8,9,10-tetrahydro-benzo[c][1,8]naphthyridin-6(5H)-on;
5-(3-Chlorphenyl)-7,8,9,10-tetrahydrobenzo[c][1,8)naphthyridin-6(5H)-on;
7-Phenyl-1,2,3,4-tetrahydro-oxepino[2,3-c][1,8]naphthyridin-6(7H)-on;
7-(3-Chlorphenyl)-1,2,3,4-tetrahydro-oxepino[2,3-c][1,8]naphthyridin-6(7H)-on;
2,3-Dihydro-6-phenyl-1H-pyrano[2,3-c][1,8]naphthyridin-5(6H)-on;
1,2-Dihydro-5-phenylfuro[2,3-c][1,8]naphthyridin-4(5H)-on; oder
1,2-Dihydro-2-(hydroxymethyl)-4-phenylcyclobuta[c][1,8]naphthyridin-3(4H)-on,
5-Phenyl-8,9-dihydro-7H-cyclopenta[c][1,8]naphthyridin-6(5H)-on• 1/4 $H_2O$.

**13.** Verwendung einer Verbindung der Formel I, definiert in irgendeinem der Ansprüche 7 bis 12, bei der Herstellung einer pharmazeutischen Zusammensetzung zur Behandlung allergischer Reaktionen bei einem Säuger.

**14.** Verwendung einer Verbindung der Formel I, definiert in irgendeinem der Ansprüche 7 bis 12, bei der Herstellung einer pharmazeutischen Zusammensetzung zur Behandlung von Entzündungen bei einem Säuger.

**15.** Verwendung einer Verbindung der Formel I, definiert in irgendeinem der Ansprüche 7 bis 12, bei der Herstellung einer pharmazeutischen Zusammensetzung zur Behandlung peptischer Geschwüre bei einem Säuger.

**16.** Verwendung einer Verbindung der Formel I, definiert in irgendeinem der Ansprüche 7 bis 12, bei der Herstellung einer pharmazeutischen Zusammensetzung zur Behandlung hyperproliferativer Hautkrankheiten bei einem Säuger.

**17.** Verwendung einer Verbindung der Formel I, definiert in irgendeinem der Ansprüche 7 bis 12, bei der Herstellung einer pharmazeutischen Zusammensetzung zur Unterdrückung der Immunantwort in einem Säuger.

**18.** Verfahren zur Herstellung einer Verbindung der Formel I

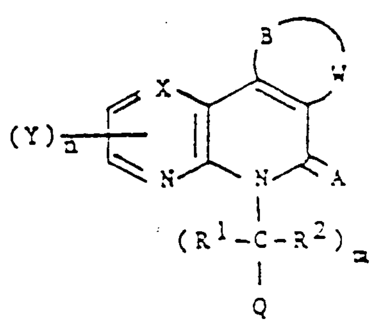

I

gekennzeichnet durch

a) Behandeln der Verbindung der Formel IV

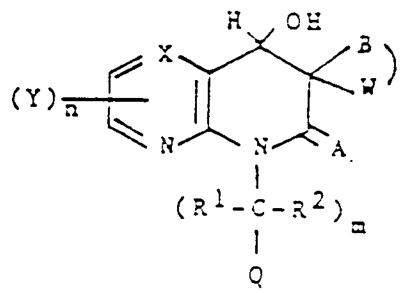

IV

mit einer Supersäure, die eine Hammett-Säurefunktion von weniger als minus 12 aufweist, um eine Verbindung der Formel I zu bilden,

b) Zusammenbringen einer Verbindung der Formel

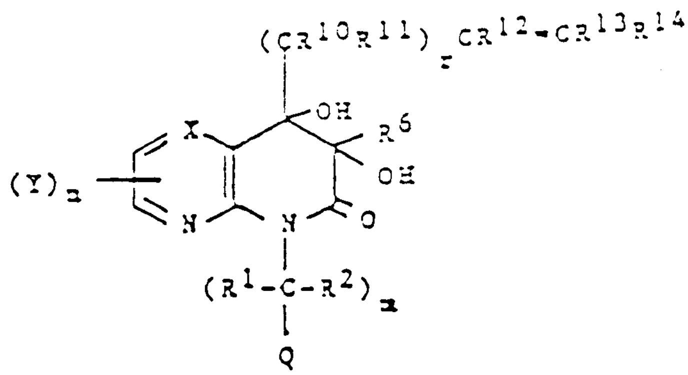

XI.

mit einer starken organischen oder anorganischen Säure, um die Verbindung I zu bilden;

c) Zusammenbringen einer Verbindung der Formel

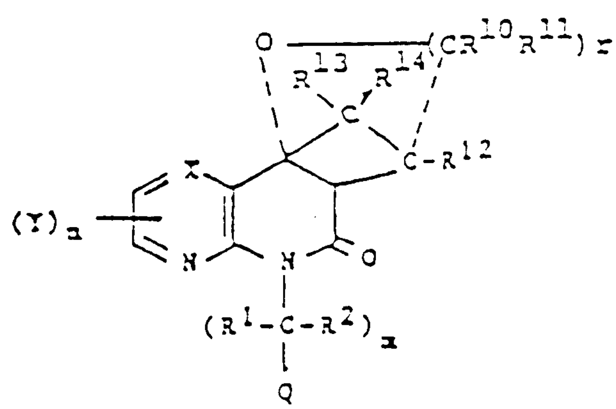

mit einer starken Base, um eine Verbindung der Formel I zu bilden; oder

d) Zusammenbringen einer Verbindung der Formel

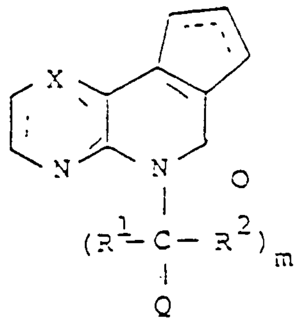

mit einem Reduktionsmittel, um eine Verbindung der Formel

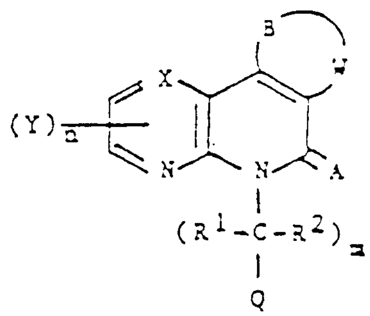

I

zu erzeugen, worin X, A, m, n, W, B, Q, Y, $R^1$, $R^2$ wie in Anspruch 1 definiert sind, und wobei $R^6$ eine Alkyl-Gruppe mit 2 bis 8 Kohlenstoff-Atomen ist; $R^{10}$, $R^{11}$, $R^{12}$, $R^{13}$ und $R^{14}$ unabhängig H oder Alkyl mit 1 bis 4 Kohlenstoff-Atomen sind; und r 0 oder 1 ist.

**Patentansprüche für folgende Vertragsstaaten : AT, ES, GR**

**1.** Verfahren zur Herstellung einer Verbindung mit der Strukturformel I

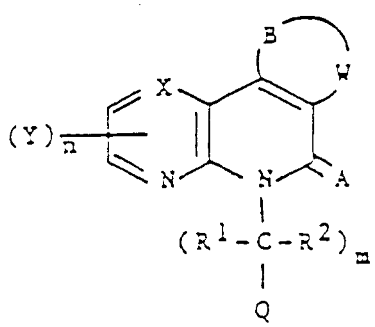

I

und deren pharmazeutisch annehmbarer Salze, worin:

| | |
|---|---|
| X | CH oder N bedeutet; |
| A | O oder S bedeutet; |
| m | eine ganze Zahl von 0 bis 2 ist; |
| n | eine ganze Zahl von 0 bis 2 ist; |
| $R^1$ und $R^2$ | gleich oder verschieden sind und jeweils unabhängig ausgewählt sind aus H oder $C_{1-6}$-Alkyl; |
| W | eine covalente Bindung bedeutet oder eine Gruppe, die ausgewählt ist aus -O-, -S(O)$_p$-, -N($R^4$)-, -NH-, -N(CO$R^4$)- und -N($SO_2R^4$)- (worin p eine ganze Zahl von 0 bis 2 und $R^4$ $C_{1-6}$-Alkyl ist); |

B Alkylen mit 2 bis 8 Kohlenstoff-Atomen bedeutet, wobei das Alkylen gegebenenfalls mit einer Gruppe substituiert sein kann, ausgewählt aus -OH, -F, Alkyl mit 1 bis 4 Kohlenstoff-Atomen, $-CH_2OH$, -CHO, $-CO_2H$, $-COR^3$ {worin $R^3$ ausgewählt ist aus $-NHR^4$, $-N(R^4)_2$ und $-OR^4$, und $R^4$ wie oben definiert ist} und -CN, mit der Maßgabe, daß OH oder F sich nicht an dem W benachbarten Kohlenstoff-Atom befindet, wenn W -O-, $-S(O)_p-$, -NH-, $-N(R^4)-$, $-N(COR^4)-$ oder $-N(SO_2R^4)-$ ist, wobei B auch ein unsubstituiertes Alkylen mit einer oder mehreren Doppelbindungen sein kann;

Q eine $C_{6-15}$-Aryl-Gruppe bedeutet oder eine aromatische heterocyclische Gruppe, die 3 bis 14 Kohlenstoff-Atome und wenigstens ein O, S und/oder N in der Ringstruktur enthält und gegebenenfalls substituiert sein kann mit bis zu drei Substituenten Y wie nachstehend definiert; und jeder Substituent Y unabhängig ausgewählt ist aus -OH, Hydroxymethyl, $C_{1-6}$-Alkyl, Halogen, $-NO_2$, $C_{1-6}$-Alkoxy, $-CF_3$, -CN, $C_{3-7}$-Cycloalkyl, $C_{3-6}$-Alkenyloxy, $C_{3-6}$-Alkinyloxy, $-S(O)_p-R^4$ (worin $R^4$ und p wie vorstehend definiert sind), $-CO-R^5$ {worin $R^5$ -OH, $-NH_2$, $-NHR^4$, $-N(R^4)_2$ oder $-OR^4$ bedeutet, worin $R^4$ wie oben definiert ist}, $-O-D-COR^5$ {worin D Alkylen mit 1 bis 4 Kohlenstoff-Atomen bedeutet und $R^5$ wie oben definiert ist}, $-NH_2$, $-NHR^4$, $-N(R^4)_2$ (worin $R^4$ wie oben definiert ist) und NHCOH,

mit der Maßgabe, daß wenn

A = O und
B = Alkylen mit 4 Kohlenstoff-Atomen und
W = eine covalente Bindung und
Q = unsubstituiertes $C_{6-15}$-Aryl,
X dann N bedeutet,

gekennzeichnet durch

a) Behandeln der Verbindung der Formel IV

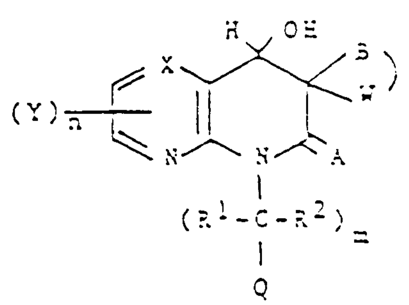

IV

mit einer Supersäure, die eine Hammett-Säurefunktion von weniger als minus 12 aufweist, um eine Verbindung der Formel I zu bilden,

b) Zusammenbringen einer Verbindung der Formel

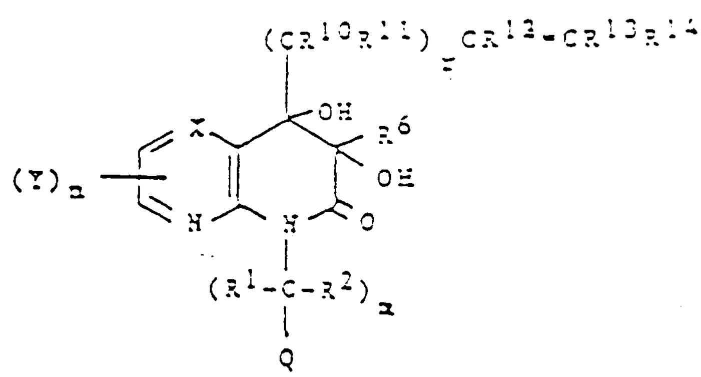

XI.

mit einer starken organischen oder anorganischen Säure, um die Verbindung I zu bilden;

c) Zusammenbringen einer Verbindung der Formel

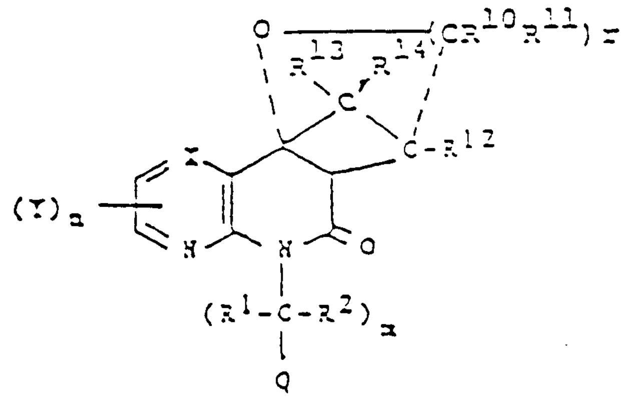

mit einer starken Base, um eine Verbindung der Formel I zu bilden; oder

d) Zusammenbringen einer Verbindung der Formel

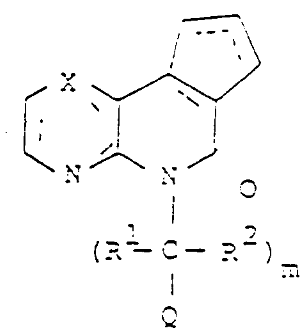

mit einem Reduktionsmittel, um eine Verbindung der Formel

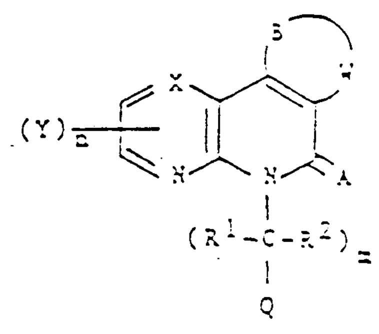

zu erzeugen, worin X, A, m, n, W, B, Q, Y, $R^1$, $R^2$ wie vorstehend definiert, und wobei $R^6$ eine Alkyl-Gruppe mit 2 bis 8 KohlenstoffAtomen ist; $R^{10}$, $R^{11}$, $R^{12}$, $R^{13}$ und $R^{14}$ unabhängig H oder Alkyl mit 1 bis 4 Kohlenstoff-Atomen sind; und r 0 oder 1 ist.

2. Verfahren nach Anspruch 1, wobei die gebildete Verbindung die Formel

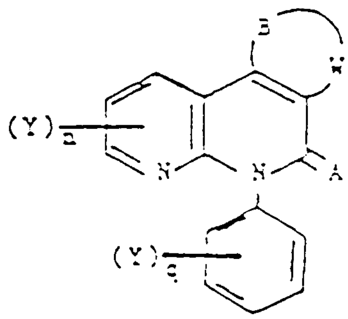

II

aufweist, worin A, B, W, Y und n wie in Anspruch 1 definiert sind, und q 0 bis 2 ist, und worin, falls n + q größer als 1 ist, die Y-Gruppen gleich oder verschieden sein können.

3. Verfahren nach Anspruch 1 oder 2, wobei -B-W- eine Alkylen-Gruppe oder eine Alkylenoxy-Gruppe bedeutet, die beide substituiert sein können.

4. Verfahren nach irgendeinem der vorstehenden Ansprüche 1 bis 3, wobei B-W $-(CH_2)_4-$, $-(CH_2)_5-$, $-(CH_2)_4-O-$ oder $-(CH_2)_3-O-$ bedeutet, und n null ist.

5. Verfahren nach irgendeinem der vorstehenden Ansprüche 1 bis 4, wobei A Sauerstoff ist, und die Y-Substituenten am Phenyl-Ring jeweils unabhängig Chlor, Nitro oder Trifluormethyl sind.

6. Verfahren nach Anspruch 1, wobei die gebildete Verbindung ausgewählt aus
5-Phenyl-7,8,9,10-tetrahydro-benzo[c][1,8]naphthyridin-6(5H)-on;
5-(3-Chlorphenyl)-7,8,9,10-tetrahydrobenzo[c][1,8]naphthyridin-6(5H)-on;
7-Phenyl-1,2,3,4-tetrahydro-oxepino[2,3-c][1,8]naphthyridin-6(7H)-on;
7-(3-Chlorphenyl)-1,2,3,4-tetrahydro-oxepino[2,3-c][1,8]naphthyridin-6(7H)-on;
2,3-Dihydro-6-phenyl-1H-pyrano[2,3-c][1,8]naphthyridin-5(6H)-on;
1,2-Dihydro-5-phenylfuro[2,3-c][1,8]naphthyridin-4(5H)-on; oder
1,2-Dihydro-2-(hydroxymethyl)-4-phenylcyclobuta[c][1,8]naphthyridin-3(4H)-on,
5-Phenyl-8,9-dihydro-7H-cyclopenta[c][1,8]naphthyridin-6(5H)-on • 1/4 $H_2O$.

7. Verfahren zur Herstellung einer pharmazeutischen Zusammensetzung, umfassend das Mischen einer Verbindung mit der Strukturformel

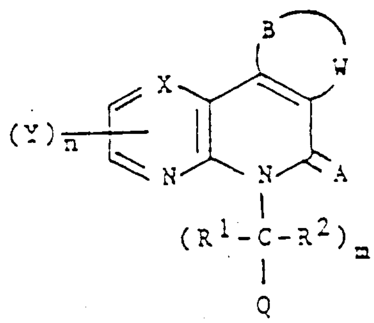

I

und pharmazeutisch annehmbarer Salze derselben, worin:

| | |
|---|---|
| X | CH oder N bedeutet; |
| A | O oder S bedeutet; |
| m | eine ganze Zahl von 0 bis 2 ist; |
| n | eine ganze Zahl von 0 bis 2 ist; |
| $R^1$ und $R^2$ | gleich oder verschieden sind und jeweils unabhängig ausgewählt sind aus H oder $C_{1-6}$-Alkyl; |

W eine covalente Bindung bedeutet oder eine Gruppe, die ausgewählt ist aus -O-, -S(O)$_p$-, -N($R^4$)-, -NH-, -N($COR^4$)- und -N($SO_2R^4$)- (worin p eine ganze Zahl von 0 bis 2 und $R^4$ $C_{1-6}$-Alkyl ist);

B Alkylen mit 2 bis 8 Kohlenstoff-Atomen bedeutet, wobei das Alkylen gegebenenfalls mit einer Gruppe substituiert sein kann, ausgewählt aus -OH, -F, Alkyl mit 1 bis 4 Kohlenstoff-Atomen, -$CH_2OH$, -CHO, -$CO_2H$, -$COR^3$ {worin $R^3$ ausgewählt ist aus -$NHR^4$, -N($R^4$)$_2$ und -$OR^4$, und $R^4$ wie oben definiert ist} und -CN, mit der Maßgabe, daß OH oder F sich nicht an dem W benachbarten Kohlenstoff-Atom befindet, wenn W -O-, -S(O)$_p$-, -NH-, -N($R^4$)-, -N($COR^4$)- oder -N($SO_2R^4$)- ist, wobei B auch ein unsubstituiertes Alkylen mit einer oder mehreren Doppelbindungen sein kann;

Q eine $C_{6-15}$-Aryl-Gruppe bedeutet oder eine aromatische heterocyclische Gruppe, die 3 bis 14 Kohlenstoff-Atome und wenigstens ein O, S und/oder N in der Ringstruktur enthält und gegebenenfalls substituiert sein kann mit bis zu drei Substituenten Y wie nachstehend definiert; und jeder Substituent Y unabhängig ausgewählt ist aus -OH, Hydroxymethyl, $C_{1-6}$-Alkyl, Halogen, -$NO_2$, $C_{1-6}$-Alkoxy, -$CF_3$, -CN, $C_{3-7}$-Cycloalkyl, $C_{3-6}$-Alkenyloxy, $C_{3-6}$-Alkinyloxy, -S(O)$_p$-$R^4$ (worin R und p wie vorstehend definiert sind), -CO-$R^5$ {worin $R^5$ -OH, -$NH_2$, -$NHR^4$, -N($R^4$)$_2$ oder -$OR^4$ bedeutet, worin $R^4$ wie oben definiert ist}, -O-D-$COR^5$ {worin D Alkylen mit 1 bis 4 Kohlenstoff-Atomen bedeutet und $R^5$ wie oben definiert ist}, -$NH_2$, -$NHR^4$, -N($R^4$)$_2$ (worin $R^4$ wie oben definiert ist) und NHCOH,

mit einem pharmazeutisch annehmbaren Träger.

**8.** Verfahren nach Anspruch 7, wobei die Verbindung die Formel

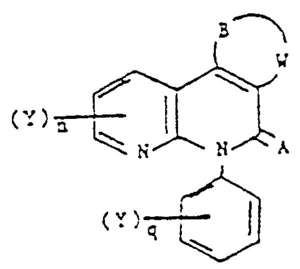

aufweist, worin A, B, W, Y und n wie in Anspruch 1 definiert sind, und q 0 bis 2 ist, und worin, falls n + g größer ist als 1, die Y-Gruppen gleich oder verschieden sein können.

**9.** Verfahren nach Anspruch 7 oder 8, wobei -B-W- eine Alkylen-Gruppe oder eine Alkylenoxy-Gruppe bedeutet, die beide substituiert sein können.

**10.** Verfahren nach irgendeinem der Ansprüche 7 bis 9, wobei B-W -$(CH_2)_4$-, -$(CH_2)_5$-, -$(CH_2)_4$-O- oder -$(CH_2)_3$-O-bedeutet, und n null ist.

**11.** Verfahren nach irgendeinem der Ansprüche 7 bis 10, wobei A Sauerstoff ist und die Y-Substituenten am Phenyl-Ring jeweils unabhängig Chlor, Nitro oder Trifluormethyl sind.

**12.** Verfahren nach Anspruch 7, wobei die Verbindung ausgewählt ist aus
5-Phenyl-7,8,9,10-tetrahydro-benzo[c][1,8]naphthyridin-6(5H)-on;
5-(3-Chlorphenyl)-7,8,9,10-tetrahydrobenzo[c][1,8]naphthyridin-6(5H)-on;
7-Phenyl-1,2,3,4-tetrahydro-oxepino[2,3-c][1,8]naphthyridin-6(7H)-on;
7-(3-Chlorphenyl)-1,2,3,4-tetrahydro-oxepino[2,3-c][1,8]naphthyridin-6(7H)-on;
2,3-Dihydro-6-phenyl-1H-pyrano[2,3-c][1,8]naphthyridin-5(6H)-on;
1,2-Dihydro-5-phenylfuro[2,3-c][1,8]naphthyridin-4(5H)-on; oder
1,2-Dihydro-2-(hydroxymethyl)-4-phenylcyclobuta[c][1,8]naphthyridin-3(4H)-on,
5-Phenyl-8,9-dihydro-7H-cyclopenta[c][1,8]naphthyridin-6(5H)-on • 1/4 $H_2O$.

**13.** Verwendung einer Verbindung der Formel I, definiert in irgendeinem der Ansprüche 7 bis 12, bei der Herstellung einer pharmazeutischen Zusammensetzung zur Behandlung allergischer Reaktionen bei einem Säuger.

**14.** Verwendung einer Verbindung der Formel I, definiert in irgendeinem der Ansprüche 7 bis 12, bei der Herstellung einer pharmazeutischen Zusammensetzung zur Behandlung von Entzündungen bei einem Säuger.

**15.** Verwendung einer Verbindung der Formel I, definiert in irgendeinem der Ansprüche 7 bis 12, bei der Herstellung einer pharmazeutischen Zusammensetzung zur Behandlung peptischer Geschwüre bei einem Säuger.

**16.** Verwendung einer Verbindung der Formel I, definiert in irgendeinem der Ansprüche 7 bis 12, bei der Herstellung einer pharmazeutischen Zusammensetzung zur Behandlung hyperproliferativer Hautkrankheiten bei einem Säuger.

**17.** Verwendung einer Verbindung der Formel I, definiert in irgendeinem der Ansprüche 7 bis 12, bei der Herstellung einer pharmazeutischen Zusammensetzung zur Unterdrückung der Immunantwort in einem Säuger.

**Revendications**
**Revendications pour les Etats contractants suivants : BE, CH, DE, FR, GB, IT, LI, LU, NL, SE**

**1.** Composé ayant la formule de structure I

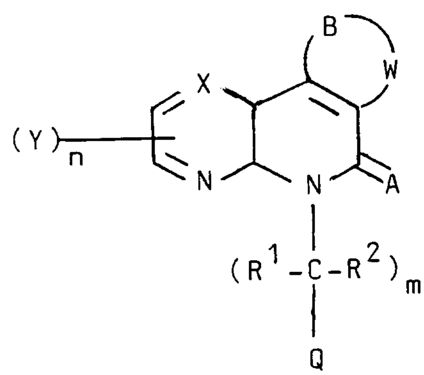

I

et ses sels acceptables en pharmacie, où :

X représente CH ou N ;
A représente O ou S ;
m est un nombre entier de 0 à 2 ;
n est un nombre entier de 0 à 2 ;

$R^1$ et $R^2$ sont identiques ou différents et chacun est indépendamment choisi parmi H ou alkyle $C_{1-6}$ ;
W représente une liaison covalente ou un groupe choisi parmi -O-, $-S(O)_p-$, $-N(R^4)-$, -NH-, $-N(COR^4)-$ et $-N(SO_2R^4)-${où p est un nombre entier de 0 à 2 et $R^4$ est alkyle $C_{1-6}$} ;
B représente un alkylène ayant 2 à 8 atomes de carbone, lequel alkylène peut être facultativement substitué par un groupe choisi parmi -OH, -F, alkyle ayant 1 à 4 atomes de carbone, $-CH_2OH$, -CHO, $-CO_2H$, $-COR^3$ {où $R^3$ est choisi parmi $-NHR^4$, $-N(R^4)_2$ et $-OR^4$ et $R^4$ est tel que défini ci-dessus} , et -CN, à condition que OH ou F ne soit pas sur le carbone adjacent à W lorsque W est -O-, $-S(O)_p$-,NH-, $-N(R^4)-$, $-N(COR^4)$ ou $-N(SO_2R^4)-$ ; B peut également représenter un alkylène non substitué ayant une ou plusieurs doubles liaisons ;
Q représente un groupe aryle $C_{6-15}$ ou un groupe hétérocyclique aromatique contenant 3 à 14 atomes de carbone et au moins un O, S et/ou N dans la structure du noyau, qui peut facultativement être substitué par jusqu'à trois substituants Y tels que définis ci-dessous ; et
chaque substituant Y est indépendamment choisi parmi -OH, hydroxyméthyle, alkyle $C_{1-6}$, halo, $-NO_2$, alcoxy $C_{1-6}$, $-CF_3$, -CN, cycloalkyle $C_{3-7}$, alkényloxy $C_{3-6}$, alkynyloxy $C_{3-6}$, $-S(O)_p-R^4$ {où $R^4$ et p

sont tels que définis ci-dessus} , -CO-$R^5$ {où $R^5$ représente -OH, -$NH_2$, -$NHR^4$, -$N(R^4)_2$ ou -$OR^4$ où $R^4$ est tel que défini ci-dessus} , -O-D-$COR^5$ {où D représente un alkylène ayant 1 à 4 atomes de carbone et $R^5$ est tel que défini ci-dessus} , -$NH_2$, - $NHR^4$, -$N(R^4)_2$ {où $R^4$ est tel que défini ci-dessus} et -NHCOH,

à condition que quand

A = 0 et

B = alkylène ayant 4 atomes de carbone et

W = une liaison covalente et

Q = aryle $C_{6-15}$ non substitué,

alors X représente N.

**2.** Composé de la revendication 1 ayant la formule

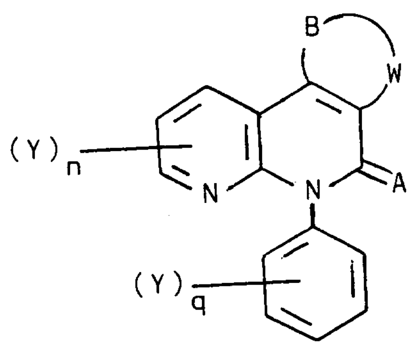

II

où A, B, W, Y et n sont tels que définis à la revendication 1 et q est 0 à 2 et où, si n+q est plus important que 1, les groupes Y peuvent être identiques ou différents.

**3.** Composé selon l'une quelconque des revendications 1 ou 2 où :
-B-W- représente un groupe alkylène ou un groupe alkylèneoxy dont chacun peut être substitué.

**4.** Composé selon l'une quelconque des revendications 1-3 ci-dessus où
B-W représente -$(CH_2)_4$-, -$(CH_2)_5$-, $(CH_2)_4$-O- ou $(CH_2)_3$-O ; et
n est zéro.

**5.** Composé selon l'une quelconque des revendications 1-4 ci-dessus où :
A est oxygène et les substituants Y sur le noyau phényle sont indépendamment chloro, nitro ou trifluorométhyle.

**6.** Composé selon la revendication 1 choisi parmi

5-phényl-7,8,9,10-tétrahydro-benzo[c][1,8]naphtyridin-6(5H)-one ;
5-(3-chlorophényl)-7,8,9,10-tétrahydrobenzo[c][1,8]naphtyridin-6(5H)-one ;
7-phényl-1,2,3,4-tétrahydro-oxépino[2,3-c][1,8]naphtyridin-6(7H)-one ;
7-(3-chlorophényl)-1,2,3,4-tétrahydro-oxépino[2,3-c][1,8]naphtyridin-6(7H)-one ;
2,3-dipydro-6-phényl-1H-pyrano[2,3-c][1,8]naphtyridin-5(6H)-one ;
1,2-dihydro-5-phénylfuro[2,3-c][1,8]naphtyridin-4(5H)-one ; ou
1,2-dihydro-2-(hydroxyméthyl)-4-phényl-cyclobuta[c][1,8]naphtyridin-3(4H)-one ;
5-phényl-8,9-dihydro-7H-cyclopenta[c][1,8]naphtyridin-6(5H)-one 1/4 $H_2O$.

7. Composition pharmaceutique qui comprend un composé ayant la formule de structure I

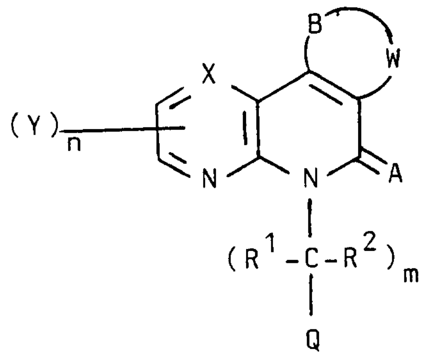

I

et ses sels acceptables en pharmacie où :

X représente CH ou N ;
A représente O ou S ;
m est un nombre entier de 0 à 2 ;
n est un nombre entier de 0 à 2 ;

$R^1$ et $R^2$ sont identiques ou différents et chacun est indépendamment choisi parmi H ou alkyle $C_{1-6}$ ;
W représente une liaison covalente ou un groupe choisi parmi -O-, $-S(O)_p-$, $-N(R^4)-$, -NH-, $-N(COR^4)-$ et $-N(SO_2R^4)-${où p est un nombre entier de 0 à 2 et $R^4$ est alkyle $C_{1-6}$} ;
B représente un alkylène ayant 2 à 8 atomes de carbone, lequel alkylène peut être facultativement substitué par un groupe choisi parmi -OH, -F, alkyle ayant 1 à 4 atomes de carbone, $-CH_2OH$, -CHO, $-CO_2H$, $-COR^3$ {où $R_3$ est choisi parmi $-NHR^4$, $-N(R^4)_2$ et $OR^4$ et $R^4$ est tel que défini ci-dessus} et -CN, à condition que OH ou F ne soient pas sur le carbone adjacent à W quand W est -O-, $-S(O)_p-$, -NH-, $-N(R^4)-$, $-N(COR^4)$ ou $-N(SO_2R^4)-$ ; B peut également représenter un alkylène non substitué ayant une ou plusieurs doubles liaisons ;
Q représente un groupe aryle $C_{6-15}$ ou un groupe aromatique hétérocyclique contenant 3 à 14 atomes de carbone et au moins l'un de O, S et/ou N dans la structure du noyau, qui peut facultativement être substitué par jusqu'à 3 substituants Y comme définis ci-dessous ; et
chaque substituant Y est indépendamment choisi parmi -OH, hydroxyméthyle, alkyle $C_{1-6}$, halo, $-NO_2$, alcoxy $C_{1-6}$, $-CF_3$, -CN, cycloalkyle $C_{3-7}$, alkényloxy $C_{3-6}$, alkynyloxy $C_{3-6}$, $-S(O)_p-R^4$ {où $R^4$ et p sont tels que définis ci-dessus} , $-CO-R^5$ {où $R^5$ représente -OH, $-NH_2$, $-NHR^4$, $-N(R^4)_2$ ou $-OR^4$ où $R^4$ est tel que défini ci-dessus} , $-O-D-COR^5$ {où D représente un alkylène ayant 1 à 4 atomes de carbone et $R^5$ est tel que défini ci-dessus} , $-NH_2$, $-NHR^4$, $-N(R^4)_2$ {où $R^4$ est tel que défini ci-dessus} et -NHCOH, en combinaison avec un support acceptable en pharmacie.

8. Composition selon la revendication 7, où ledit composé a pour formule

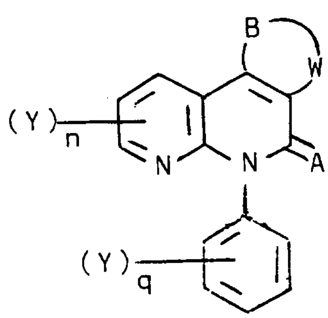

II

où A, B, W, Y et n sont tels que définis à la revendication 1 et Q est 0 à 2 et où, si n+q est plus important que 1, les groupes Y peuvent être identiques ou différents.

**9.** Composition selon la revendication 7 ou la revendication 8 où -B-W- représente un groupe alkylène ou un groupe alkylèneoxy dont chacun peut être substitué.

**10.** Composition selon l'une quelconque des revendications 7 à 9 où B-W représente $-(CH_2)_4-$, $-(CH_2)_5-$, $(CH_2)_4-O-$ ou $(CH_2)_3-O$ ; et n est zéro.

**11.** Composition selon l'une quelconque des revendications 7 à 10, où A est oxygène et les substituants Y sur le noyau phényle sont indépendamment chloro, nitro ou trifluorométhyle.

**12.** Composition selon la revendication 7 où ledit composé est choisi parmi

5-phényl-7,8,9,10-tétrahydro-benzo[c][1,8]naphtyridin-6(5H)-one ;
5-(3-chlorophényl)-7,8,9,10-tétrahydro-benzo[c][1,8]naphtyridin-6(5H)-one ;
7-phényl-1,2,3,4-tétrahydro-oxépino[2,3-c][1,8]naphtyridin-6(7H)-one ;
7-(3-chlorophényl)-1,2,3,4-tétrahydro-oxépino[2,3-c][1,8]naphtyridin-6(7H)-one ;
2,3-dihydro-6-phényl-1H-pyrano[2,3-c][1,8]naphtyridin-5(6H)-one ;
1,2-dihydro-5-phénylfuro[2,3-c][1,8]naphtyridin-4(5H)-one ; ou
1,2-dihydro-2-(hydroxyméthyl)-4-phényl-cyclobuta[c][1,8]naphtyridin-3(4H)-one ;
5-phényl-8,9-dihydro-7H-cyclopenta[c][1,8]naphtyridin-6(5H)-one 1/4 $H_2O$.

**13.** Utilisation d'un composé de formule I défini selon l'une quelconque des revendications 7 à 12 dans la fabrication d'une composition pharmaceutique pour le traitement des réactions allergiques chez un mammifère.

**14.** Utilisation d'un composé de formule I défini selon l'une quelconque des revendications 7 à 12 dans la fabrication d'une composition pharmaceutique pour le traitement d'une inflammation chez un mammifère.

**15.** Utilisation d'un composé de formule I défini selon l'une quelconque des revendications 7 à 12 dans la fabrication d'une composition pharmaceutique pour le traitement des ulcères simples de l'estomac chez un mammifère.

**16.** Utilisation d'un composé de formule I défini selon l'une quelconque des revendications 7 à 12 dans la fabrication d'une composition pharmaceutique pour le traitement d'une maladie hyperproliférative de la peau chez un mammifère.

**17.** Utilisation d'un composé de formule I défini selon l'une quelconque des revendications 7 à 12 dans la fabrication d'une composition pharmaceutique pour supprimer la réponse immunitaire d'un animal.

**18.** Procédé de préparation d'un composé de formule I

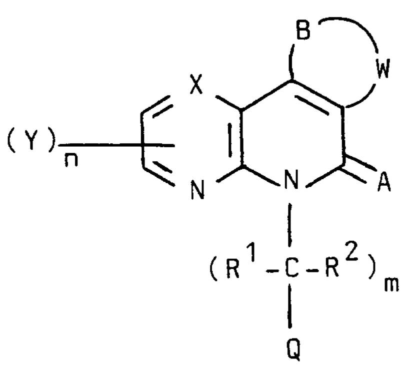

I

ledit procédé étant caractérisé par A.

a) le traitement du composé de formule IV

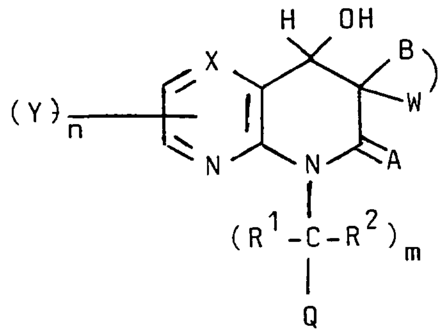

IV

avec un super-acide ayant une fonction d'acidité de Hammett de moins de moins 12, pour former un composé de formule I,

b) la mise en contact d'un composé de la formule

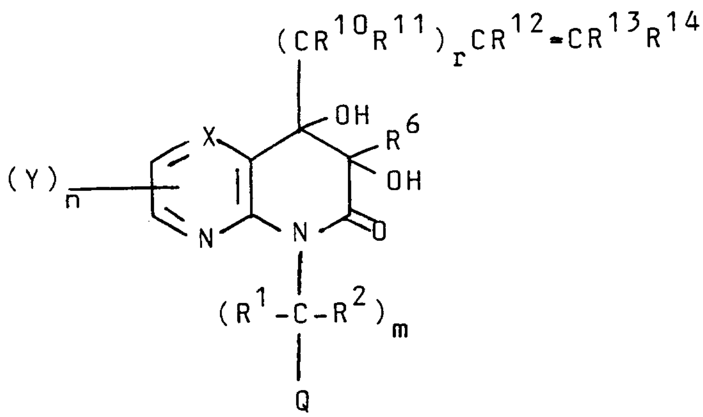

XI

avec un acide organique ou inorganique fort pour donner le composé I ;

c) la mise en contact d'un composé de formule

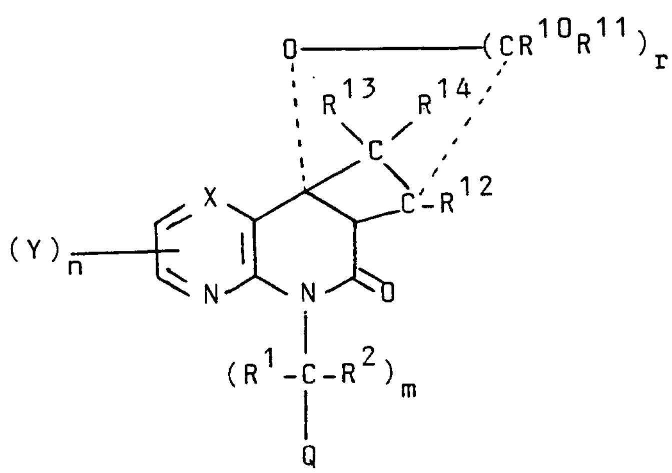

avec une base forte pour former un composé de formule I ;

d) la mise en contact d'un composé de formule

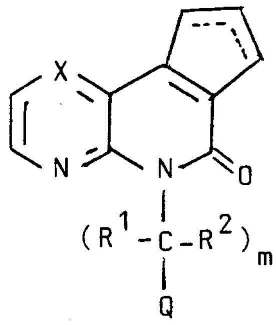

avec un agent réducteur pour donner un composé de la formule

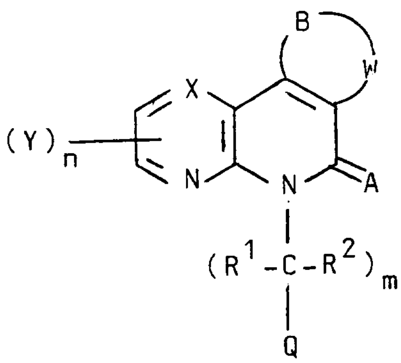

I

où X, A, m, n, W, B, Q, Y, $R^1$ et $R^2$ sont tels que définis à la revendication 1 et où

$R^6$ est un groupe alkyle ayant 2 à 8 atomes de carbone ;

$R^{10}$, $R^{11}$ , $R^{12}$, $R^{13}$ et $R^{14}$ sont indépendamment H ou un alkyle ayant 1 à 4 atomes de carbone ;

et

r est 0 ou 1.

**Revendications pour les Etats contractants suivants : AT, ES, GR**

**1.** Procédé de production d'un composé ayant la formule de structure I

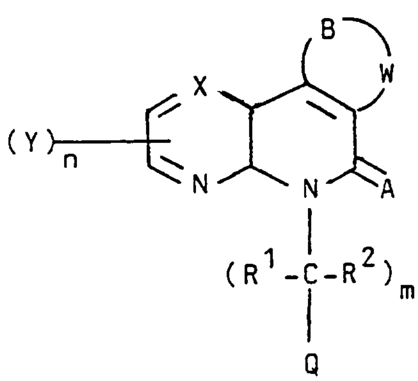

I

et ses sels acceptables en pharmacie, où :

X représente CH ou N ;

A représente O ou S ;

m est un nombre entier de 0 à 2 ;

n est un nombre entier de 0 à 2 ;
$R^1$ et $R^2$ sont identiques ou différents et chacun est indépendamment choisi parmi H ou alkyle $C_{1-6}$ ;
W représente une liaison covalente ou un groupe choisi parmi -O-, $-S(O)_p-$, $-N(R^4)-$, -NH-, $-N(COR^4)-$ et $-N(SO_2R^4)-$ {où p est un nombre entier de 0 à 2 et $R^4$ est alkyle $C_{1-6}$} ;
B représente un alkylène ayant 2 à 8 atomes de carbone, lequel alkylène peut être facultativement substitué par un groupe choisi parmi -OH, -F, alkyle ayant 1 à 4 atomes de carbone, $-CH_2OH$, -CHO, $-CO_2H$, $-COR^3$ {où $R^3$ est choisi parmi $-NHR^4$, $-N(R^4)_2$ et $-OR^4$ et $R^4$ est tel que défini ci-dessus}, et -CN, à condition que OH ou F ne soit pas sur le carbone adjacent à W lorsque W est -O-, $-S(O)_p-$,NH-, $-N(R^4)-$, $-M(COR^4)$ ou $-N(SO_2R^4)-$ ; B peut également représenter un alkylène non substitué ayant une ou plusieurs doubles liaisons ;
Q représente un groupe aryle $C_{6-15}$ ou un groupe hétérocyclique aromatique contenant 3 à 14 atomes de carbone et au moins un O, S et/ou N dans la structure du noyau, qui peut facultativement être substitué par jusqu'à trois substituants Y tels que définis ci-dessous ; et
chaque substituant Y est indépendamment choisi parmi -OH, hydroxyméthyle, alkyle $C_{1-6}$, halo, $-NO_2$, alcoxy $C_{1-6}$, $-CF_3$, -CN, cycloalkyle $C_{3-7}$, alkényloxy $C_{3-6}$, alkynyloxy $C_{3-6}$, $-S(O)_p-R^4$ {où $R^4$ et p sont tels que définis ci-dessus}, $-CO-R^5$ {où $R^5$ représente -OH, $-NH_2$, $-NHR^4$, $-N(R^4)_2$ ou $-OR^4$ où $R^4$ est tel que défini ci-dessus} , $-O-D-COR^5$ {où D représente un alkylène ayant 1 à 4 atomes de carbone et $R^5$ est tel que défini ci-dessus} , $-NH_2$, - $NHR^4$, $-N(R^4)_2$ {où $R^4$ est tel que défini ci-dessus} et -NHCOH,
à condition que quand
A = O et
B = alkylène ayant 4 atomes de carbone et
W = une liaison covalente et
Q = aryle $C_{6-15}$ non substitué,
alors X représente N, ledit procédé étant caractérisé par
a) le traitement du composé de formule IV

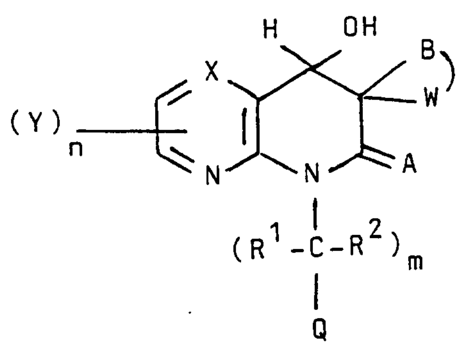

IV

avec un super-acide ayant une fonction d'acidité de Hammett de moins de moins 12, pour former un composé de formule I,

b) la mise en contact d'un composé de formule

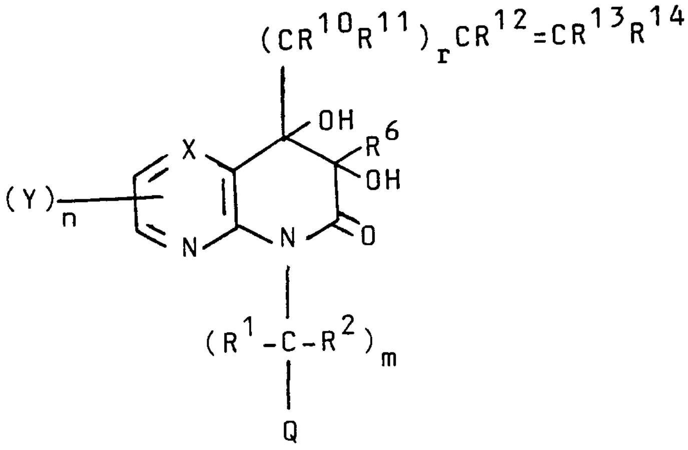

XI

avec un acide organique ou inorganique fort pour donner le composé I ;
c) la mise en contact d'un composé de formule

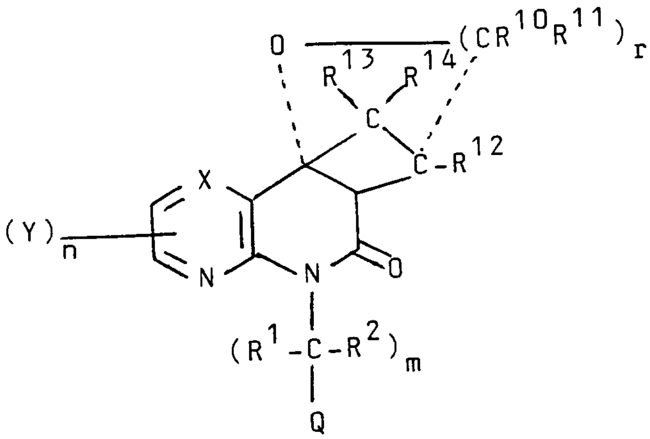

avec une base forte pour former un composé de formule I; ou
d) la mise en contact d'un composé de formule

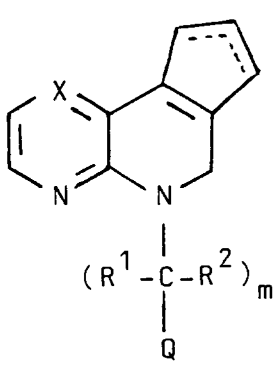

avec un agent réducteur pour donner un composé de formule

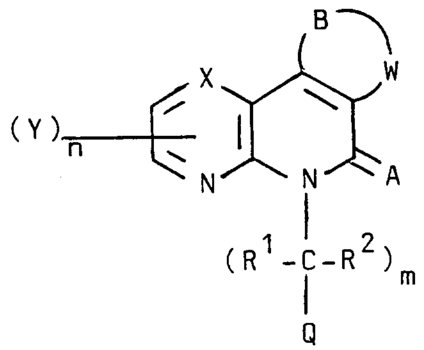

I

où X, A, m, n, W, B, Q, Y, $R^1$ et $R^2$ sont tels que précédemment définis et où $R^6$ est un groupe alkyle ayant 2 à 8 atomes de carbone ; $R^{10}$, $R^{11}$, $R^{12}$, $R^{13}$ et $R^{14}$ sont indépendamment H ou un alkyle ayant 1 à 4 atomes de carbone ; et r est 0 ou 1.

**2.** Procédé de la revendication 1 où ledit composé produit a pour formule

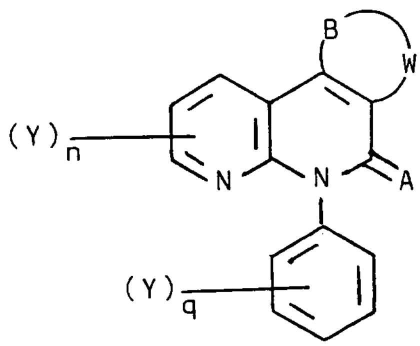

où A, B, W, Y, et n sont tels que définis à la revendication 1 et q est 0 à 2 et où, si n+q représente plus de 1, les groupes Y peuvent être identiques ou différents.

**3.** Procédé selon l'une quelconque des revendications 1 ou 2 où :
-B-W- représente un groupe alkylène ou un groupe alkylèneoxy dont chacun peut être substitué.

**4.** Procédé selon l'une quelconque des revendications 1-3 où
B-W représente $-(CH_2)_4-$, $-(CH_2)_5-$, $-(CH_2)_4-O-$ ou $(CH_2)_3-O$ ; et
n est zéro.

**5.** Procédé selon l'une quelconque des revendications 1-4 où :
A est oxygène et les substituants Y sur le noyau phényle sont indépendamment chloro, nitro ou trifluorométhyle.

**6.** Procédé selon la revendication 1 où ledit composé produit est choisi parmi
5-phényl-7,8,9,10-tétrahydro-benzo[c][1,8]naphtyridin-6(5H)-one ;
5-(3-chlorophényl)-7,8,9,10-tétrahydro-benzo[c][1,8]naphtyridin-6(5H)-one ;
7-phényl-1,2,3,4-tétrahydro-oxépino[2,3-c][1,8]naphtyridin-6(7H)-one ;
7-(3-chlorophényl)-1,2,3,4-tétrahydro-oxépino[2,3-c][1,8]naphtyridin-6(7H)-one ;
2,3-dihydro-6-phényl-1H-pyrano[2,3-c][1,8]naphtyridin-5(6H)-one ;
1,2-dihydro-5-phénylfuro[2,3-c][1,8]naphtyridin-4(5H)-one ; ou
1,2-dihydro-2-(hydroxyméthyl)-4-phényl-cyclobuta[c][1,8]naphtyridin-3(4H)-one ;
5-phényl-8,9-dihydro-7H-cyclopenta[c][1,8]naphtyridin-6(5H)-one 1/4 $H_2O$.

**7.** Méthode de production d'une composition pharmaceutique qui consiste à mélanger un composé ayant la formule de structure

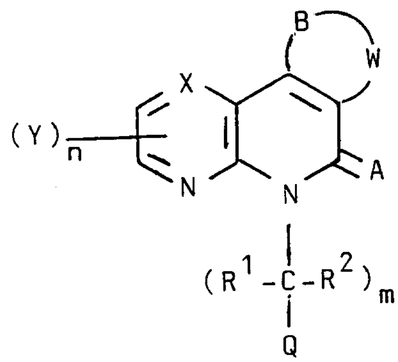

I

et ses sels acceptables en pharmacie, où

X représente CH ou N ;

A représente O ou S ;

m est un nombre entier de 0 à 2 ;

n est un nombre entier de 0 à 2 ;

$R^1$ et $R^2$ sont identiques ou différents et chacun est indépendamment choisis parmi H ou alkyle $C_{1-6}$ ;

W représente une liaison covalente ou un groupe choisi parmi -O-, $-S(O)_p-$, $-N(R^4)-$, -NH-, $-N(COR^4)-$ et $-N(SO_2R^4)-${où p est un nombre entier de 0 à 2 et $R^4$ est alkyle $C_{1-6}$};

B représente alkylène ayant 2 à 8 atomes de carbone, lequel alkylène peut être facultativement substitué par un groupe choisi parmi -OH, -F, alkyle ayant 1 à 4 atomes de carbone, $-CH_2OH$, -CHO, $-CO_2H$, $-COR^3$ {où $R^3$ est choisi parmi $-NHR^4$, $-N(R^4)_2$ et $-OR^4$ et $R^4$ est tel que défini ci-dessus} , et -CN, à condition que OH ou F ne soit pas sur le carbone adjacent à W quand W est -O-, $-S(O)_p-$,-NH-, $-N(R^4)-$, $-N(COR^4)$ ou $-N(SO_2R^4)-$ ; B peut également représenter un alkylène non substitué ayant une ou plusieurs doubles liaisons ;

Q représente un groupe aryle $C_{6-15}$ ou un groupe hétérocyclique aromatique contenant 3 à 14 atomes de carbone et au moins un O, S et/ou N dans la structure du noyau, qui peut être facultativement substitué par jusqu'à 3 substituants Y tels que définis ci-dessous ;

et

chaque substituant Y est indépendamment choisi parmi -OH, hydroxyméthyle, alkyle $C_{1-6}$, halo, $-NO_2$, alcoxy $C_{1-6}$, $-CF_3$, -CN, cycloalkyle $C_{3-7}$, alkényloxy $C_{3-6}$, alkynyloxy $C_{3-6}$, $-S(O)_p-R^4$ {où $R^4$ et p sont tels que définis ci-dessus}, $-CO-R^5$ {où $R^5$ représente -OH, $-NH_2$, $-NHR^4$, $-N(R^4)_2$ ou $-OR^4$ où $R^4$ est tel que défini ci-dessus}, $-O-D-COR^5$ {où D représente un alkylène ayant 1 à 4 atomes de carbone et $R^5$ est tel que défini ci-dessus}, $-NH_2$, $-NHR^4$, $-N(R^4)_2$ {où $R^4$ est tel que défini ci-dessus} et -NHCOH, avec un support acceptable en pharmacie.

**8.** Méthode selon la revendication 7 où ledit composé a pour formule

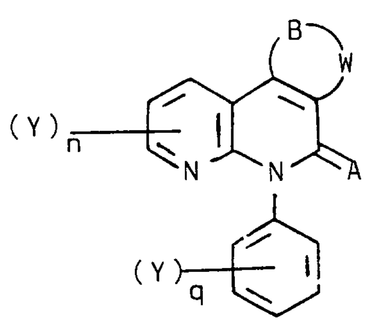

où A, B, W, Y et n sont tels que définis à la revendication 1 et q est 0 à 2 et où, si n + q est plus grand

que 1 , les groupes Y peuvent être identiques ou différents.

9. Méthode selon la revendication 7 ou la revendication 8 où -B-W- représente un groupe alkylène ou un groupe alkylèneoxy dont chacun peut être substitué.

10. Méthode selon l'une quelconque des revendications 7 à 9 où B-W représente $-(CH_2)_4-$, $-(CH_2)_5-$, $(CH_2)_4-O-$ ou $(CH_2)_3-O$ ; et n est zéro.

11. Méthode selon l'une quelconque des revendications 7 à 10 où A est oxygène et les substituants Y sur le noyau phényle sont indépendamment chloro, nitro ou trifluorométhyle.

12. Méthode selon la revendication 17 où ledit composé est choisi parmi
5-phényl-7,8,9,10-tétrahydro-benzo[c][1,8]naphtyridin-6(5H)-one ;
5-(3-chlorophényl)-7,8,9,10-tétrahydro-benzo[c][1,8]naphtyridin-6(5H)-one ;
7-phényl-1,2,3,4-tétrahydro-oxépino[2,3-c][1,8]naphtyridin-6(7H)-one ;
7-(3-chlorophényl)-1,2,3,4-tétrahydro-oxépino[2,3-c][1,8]naphtyridin-6(7H)-one ;
2,3-dihydro-6-phényl-1H-pyrano[2,3-c][1,8]naphtyridin-5(6H)-one ;
1,2-dihydro-5-phénylfuro[2,3][1,8]-naphtyridin-4(5H)-one ; ou
1,2-dihydro-2-(hydroxyméthyl)-4-phényl-cyclobuta[c][1,8]naphtyridin-3(4H)-one ;
5-phényl-8,9-dihydro-7H-cyclopenta[c][1,8]naphtyridin-6(5H)-one 1/4 $H_2O$.

13. Utilisation d'un composé de formule I défini selon l'une quelconque des revendications 7 à 12 dans la fabrication d'une composition pharmaceutique pour le traitement de réactions allergiques chez un mammifère.

14. Utilisation d'un composé de formule I défini selon l'une quelcomque des revendications 7 à 12 dans la fabrication d'une composition pharmaceutique pour le traitement de l'inflammation chez un mammifère.

15. Utilisation d'un composé de formule I défini selon l'une quelconque des revendications 7 à 12 dans la fabrication d'une composition pharmaceutique pour le traitement des ulcères simples de l'estomac chez un mammifère.

16. Utilisation d'un composé de formule I défini selon l'une quelconque des revendications 7 à 12 dans la fabrication d'une composition pharmaceutique pour le traitement d'une maladie hyperproliférative de la peau chez un mammifère.

17. Utilisation d'un composé de formule I défini selon l'une quelconque des revendications 7 à 12 dans la fabrication d'une composition pharmaceutique pour supprimer la réponse immunitaire d'un mammifère.